# EUROPEAN PATENT APPLICATION

(11) **EP 3 903 856 A1**
(43) Date of publication of application: **03.11.2021**
(21) Application number: 19906483.3
(22) Date of filing: 25.12.2019
(51) Int. Cl.: A61M 5/142, A61M 5/158

(54) **MEDICINE ADMINISTERING DEVICE AND MEDICINE ADMINISTERING SYSTEM**

(30) Priority: 25.12.2018 JP 2018241860; 13.11.2019 JP 2019205869; 11.12.2019 JP 2019224089
(71) Applicant: HIROSAKI UNIVERSITY, Hirosaki-shi, Aomori 036-8560 (JP); Otsuka Pharmaceutical Factory, Inc., Naruto-shi, Tokushima 772-8601 (JP)
(72) Inventor: KOBAYASHI, Tadashi, Hirosaki-shi, Aomori 036-8560 (JP); MAITA, Hiroki, Hirosaki-shi, Aomori 036-8560 (JP); KATO, Hiroyuki, Hirosaki-shi, Aomori 036-8560 (JP); AKIMOTO, Takashi, Hirosaki-shi, Aomori 036-8560 (JP); MISAWA, Hidetoshi, Kasama-shi, Ibaraki 309-1717 (JP); HAYASHI, Takehito, Naruto-shi, Tokushima 772-8601 (JP); TAKEDA, Koichi, Naruto-shi, Tokushima 772-8601 (JP); TERAO, Toshimitsu, Naruto-shi, Tokushima 772-8601 (JP); MORITA, Tomoki, Naruto-shi, Tokushima 772-8601 (JP); ITO, Shinichiro, Naruto-shi, Tokushima 772-8601 (JP)
(74) Representative: Winter, Brandl - Partnerschaft mbB
(86) International application number: PCT/JP2019/050883
(87) International publication number: WO 2020/138193

(57) **Abstract**

A drug administration device according to the present invention is a drug administration device for subcutaneously administering a drug, and includes a main body portion configured to be arranged on skin of a patient, and a movable portion to which at least one needle member protruding toward the skin is attached. The movable portion is configured to be capable of being moved between a first position that is spaced apart from the skin and a second position that is near the skin. The leading end portion of the needle member is to be inserted into the skin when the movable portion is located at the second position. The drug is to be discharged from a hole provided in the needle member.

## Description

### TECHNICAL FIELD

The present invention relates to a drug administration device and a drug administration system.

### BACKGROUND ART

Peripheral vein infusion therapy in which a drug contained in an infusion bag is administered to a peripheral vein of a patient via a catheter has been conventionally known. However, it may be difficult to secure a peripheral vein due to the age, disease, or state of a patient, and there is the problem of elderly persons or dementia patients, or children including infants removing the needle by themselves and bleeding.

In recent years, subcutaneous infusion therapy has been reviewed again as infusion therapy for such patients. Subcutaneous infusion therapy solves the problem of it being difficult to secure a peripheral vein, and thus it can be said that it is significantly advantageous in other respects such as safety and manageability. It is thought that products will be desired that will meet both needs and risks that need to be dealt with in current medical practice as subcutaneous infusion therapy becomes widespread in current medical practice. For example, the drug administration device disclosed in the specification of U.S. Patent No. 7150726 includes a substrate from which a plurality of needle members connected to a drug line protrude, and is configured such that this substrate is attached to the skin to insert the needle members into the skin, and thus the drug can be subcutaneously administered.

### CITATION LIST

### PATENT DOCUMENT

Patent Document 1: US 7,150,726

### SUMMARY OF THE INVENTION

### PROBLEMS TO BE SOLVED BY THE INVENTION

However, with the above-mentioned device, the needle members are inserted into the skin simultaneously with the attachment of the substrate to the skin. Accordingly, the positioning of the needles and the insertion of the needles need to be performed simultaneously, and thus the operation is complicated. There is also a safety problem because the needle members are exposed. Furthermore, it is thought that the exposed needle members cause a patient to feel a psychological barrier regarding the insertion of the needle member into the skin.

The present invention was achieved in order to solve the foregoing problems, and it is an object thereof to provide a drug administration device and a drug administration system with which subcutaneous administration of a liquid drug can be easily performed.

### MEANS FOR SOLVING THE PROBLEMS

A drug administration device according to the present invention is a drug administration device for subcutaneously administering a drug, including: a main body portion configured to be arranged on skin of a patient; and a movable portion to which at least one needle member protruding toward the skin is attached, wherein the movable portion is configured to be capable of being moved between a first position that is spaced apart from the skin and a second position that is near the skin, a leading end portion of the needle member is to be inserted into the skin when the movable portion is located at the second position, and the drug is to be discharged from a hole provided in the needle member.

With this configuration, the needle member can be inserted into the skin by moving the movable portion from the first position to the second position. The drug can be discharged through the hole provided in the needle member, and therefore, the drug can be subcutaneously administered merely by moving the movable portion to the second position. Accordingly, medical staff or patients can perform subcutaneous administration of the drug on their own with a simple operation.

It should be noted that the needle member may be directly attached to the movable portion or indirectly attached to the movable portion via another member. Moreover, the main body portion is arranged on the skin of a patient, and this encompasses a case where the main body portion is directly arranged thereon and a case where the main body portion is indirectly arranged thereon via another member such as an adhering material.

In the above-mentioned drug administration device, the movable portion can be configured to be held at at least one of the first position and the second position in a state in which an external force is not applied to the movable portion.

With this configuration, the movable portion can be kept in at least one of the state in which the needle member is inserted into the skin and the state in which the needle member is spaced apart from the skin without the need for applying an external force, and thus the handling of the device is facilitated. It should be noted that, in the case where the movable portion is configured such that it is necessary to apply an external force in order to hold the movable portion at one of the positions, the movable portion can be forcibly held at one of the positions using another jig such as a stopper.

The above-mentioned drug administration device can further include a coupling portion that couples the main body portion and the movable portion to each other and is configured to be capable of holding the movable portion at at least one of the first position and the second position. In addition, the movable portion can also be held at one of the positions using a member that is not coupled to the main body portion, for example.

Coupling the main body portion and the movable portion to each other using the coupling portion makes it possible to reduce the number of components of a final product, for example. Moreover, providing the coupling portion makes it possible to hold the movable portion at the above-mentioned position with a simple configuration.

In the above-mentioned drug administration device, the coupling portion can be variously configured. For example, the coupling portion can be configured to surround the entire perimeter of the needle member. When a plurality of coupling portions are provided, the coupling portions can be radially arranged around the needle member at predetermined intervals.

With this configuration, the coupling portions are radially arranged at predetermined intervals and can thus support the movable portion in a balanced manner in the circumferential direction. Therefore, when the movable portion is moved between the first position and the second position, it is possible to suppress a problem of the movable portion being inclined when being moved.

In the above-mentioned drug administration device, the coupling portions are formed of a material that can be elastically deformed, and are configured to enter a first state when located at the first position and enter a second state when located at the second position, the coupling portions in the second state are bent, and the coupling portions in the first state are more stretched than those in the second state, and the coupling portions can be configured to make a transition between the first state and the second state while being elastically deformed.

With this configuration, each of the coupling portions is configured to make a transition from the first state and the second state while being elastically deformed, and therefore, it is possible to hold the movable portion at the first position or the second position as long as an external force is not applied to the coupling portion and the coupling portion is not elastically deformed.

When a plurality of coupling portions are provided as described above, it is possible to provide two to ten coupling portions, for example.

In the above-mentioned drug administration device, the main body portion, the movable portion, and the coupling portion can be formed as a single body.

With this configuration, the main body portion, the movable portion, and the coupling portion can be treated as a single member, and thus the overall structure of the drug administration device can be simplified. Accordingly, the manufacturing cost can also be reduced due to a decrease in the number of components.

In the above-mentioned drug administration device, the main body portion, the movable portion, and the coupling portion can be formed of an elastically deformable elastomer, hard resin, or metal.

With this configuration, when these portions are formed of an elastomer, a good curved skin following property is achieved, and thus the needle member can be appropriately inserted into the skin. When these portions are formed of a hard resin or metal, a device that is stable against an external force can be obtained.

In the above-mentioned drug administration device, the main body portion can be formed in a tubular shape, and the movable portion can be configured such that at least a portion of the movable portion is accommodated in the main body portion when the movable portion is located at the second position.

With this configuration, when the movable portion is located at the second position, the needle member is inserted into the skin in the state in which the needle member is accommodated in the main body portion. Therefore, if an external force is applied to the drug administration device at the time of puncture, for example, an influence on the needle member can be reduced, and thus it is possible to prevent the needle from coming out or bending, or to prevent the needle member from moving in the state in which the needle member is inserted into the skin.

In the above-mentioned drug administration device, a drug line for supplying the drug can be attached, and the drug can be supplied from the drug line to the needle member.

With this configuration, a drug can be administered for a long period of time by connecting the drug line to a drug container such as an infusion bag, for example.

The above-mentioned drug administration device can further include an operation portion that is coupled to the movable portion and can be held by tools or fingers.

With this configuration, the movable portion can be easily moved from the second position to the first position by pulling the operation portion using tools or fingers, for example.

Although there is no particular limitation on the configuration of the operation portion, the operation portion can be formed in a plate shape whose external size is larger than that of the main body portion.

With this configuration, the size of the operation portion in the axial direction of the main body portion can be reduced. Increasing the size of the operation portion makes it possible to push the operation portion using a finger at the time of puncture while bringing the finger into surface contact therewith, which contributes to a stable puncture operation.

The above-mentioned drug administration device can further include an extension portion connected to the operation portion, and the extension portion can be configured to cover at least a portion of the main body portion when the movable portion is located at the second position.

With this configuration, the extension portion is configured to cover at least a portion of the main body portion when the movable portion is located at the second position, and thus the extension portion can be brought into contact with the skin or arranged near the skin. Therefore, the operation portion can be stably held at the second position. Accordingly, the operation portion protects the entire main body portion, and it is possible to prevent the movable portion from being shifted or lifted up together with the operation portion when an impact or load is applied to the operation portion in the lateral direction, for example. Thus, it is possible to prevent the needle member inserted into the skin from unexpectedly coming out, or to prevent the needle member from moving in the state in which the needle member is inserted into the skin.

In the above-mentioned drug administration device, the operation portion can be configured to protrude outward from the outer circumference of the movable portion in a radial direction, and a stopper can be further included that is detachably arranged between the operation portion and the main body portion when the movable portion is located at the first position, and restricts movement of the movable portion to the second position.

This configuration includes the stopper that can be arranged between the operation portion and the main body portion when the movable portion is located at the first position. Therefore, this stopper prevents the operation portion from being accidentally moved toward the main body portion to the second position. Thus, the movable portion can be held at the first position.

The above-mentioned drug administration device further includes a storage portion for storing the drug, and can be configured such that the drug is supplied from the storage portion to the needle member. Note that the drug can be supplied to the storage portion from the drug line.

With this configuration, a small amount of drug such as insulin can be stored in the storage portion. Then, for example, by pressing the storage portion to apply pressure when the movable portion of the drug administration device is located at the second position, the drug in the storage portion can be administered to a patient. Further, the drug can be also supplied to the storage portion from the drug line. In this case, although the drug is generally supplied from the drug line to the needle line, the drug stored in the storage portion can be discharged through the needle member in a case where the supply of the drug from the drug line is suddenly stopped for some reason, for example. This makes it possible to prevent the administration of the drug from being suspended, which can contribute to stable administration of the drug.

The above-mentioned drug administration device can further include a base that supports the main body portion and is to be arranged on the skin.

With this configuration, the drug administration device can be fixed to the skin over a large area, and thus this device can be stably fixed to the skin.

The above-mentioned drug administration device can further include an adhering material arranged on at least a portion of a surface of the base that is to be brought into contact with the skin.

With this configuration, the base can be firmly fixed to the skin using the adhering material, and thus the drug can be administered for a long period of time.

In the above-mentioned drug administration device, a recessed portion can be provided on a surface of a circumferential edge portion of the base that is to be opposite to the skin so as to form a gap formed between the base and the skin.

With this configuration, it is possible to insert a finger between the base and the skin because the recessed portion is provided, and thus the base can be easily removed from the skin.

In the above-mentioned drug administration device, a plurality of drug administration portions can be arranged on the base. The drug administration portion can include the main body portion, the movable portion, and the needle member. The drug administration portion may include, for example, the above-mentioned coupling portion, if necessary, in addition to the main body portion, the movable portion, and the needle member.

With this configuration, a large amount of a drug can be subcutaneously administered in a short period of time. Note that the drug administration portion can include the above-mentioned coupling portion.

In the above-mentioned drug administration device, all of the movable portions of the drug administration portions can be coupled to each other.

With this configuration, all of the movable portions are coupled to each other, and thus all of the movable portions of the drug administration portions can be moved from the first position to the second position or from the second position to the first position by one action. Therefore, operation can be efficiently performed. It should be noted that the movable portions can be coupled to each other using the above-described operation portion, for example.

The above-mentioned drug administration device can further include a base that supports the main body portion and is to be arranged on the skin. In this drug administration device, the base, the main body portion, the movable portion, and the coupling portion can be formed of an elastomer as a single body.

With this configuration, the base to be arranged on the skin is also formed of an elastomer and can thus be deformed so as to follow the skin surface and arranged thereon. In addition, the base is formed as a single body with the main body portion, the movable portion, and the coupling portion, and therefore, the main body portion, the movable portion, and the coupling portion can follow the deformation of the base following the skin. Accordingly, the needle member protruding from the movable portion can precisely protrude from the through hole of the base. Furthermore, even when the drug administration device is attached to the skin for a long period of time, a risk that the drug administration device will come off from the skin can be reduced. Moreover, the number of components of the device is reduced, and a member constituted by the base, the main body portion, the movable portion, and the coupling portion can be formed through a technique such as injection molding. Thus, the manufacturing cost is reduced, and discarding thereof is facilitated. In addition, the structure is further simplified due to the decrease in the number of components, and thus is easy to handle even for inexperienced users.

In the above-mentioned drug administration device, the main body portion is configured to be attached to the skin, and the main body portion can be provided with a recessed portion so as to form a gap between the main body portion and the skin.

With this configuration, it is possible to insert a finger between the main body portion and the skin because the recessed portion is provided, and thus the main body portion can be easily removed from the skin.

In the above-mentioned drug administration device, the needle member is fixed to the operation portion, and the operation portion can be detached from the movable portion together with the needle member.

With this configuration, the needle member can be detached from the movable portion by pulling the operation portion out of the main body portion after the drug is subcutaneously administered. As a result, it is possible to prevent reuse of the drug administration device and to separately collect the needle member.

The above-mentioned drug administration device can further include a detaching means for detaching the needle member from the movable portion.

With this configuration, the drug administration device is destroyed using the detaching means after subcutaneous administration of the drug is complete, and it is possible to prevent reuse of the needle member. In addition, it is possible to separately collect the needle member.

The above-mentioned drug administration device can further include a cover member that can cover at least a leading end of the needle member, and a configuration can be employed in which the leading end of the needle member is exposed from the cover member when the movable portion is located at the second position, and the cover member covers the leading end of the needle member and this covering state is maintained after the movable portion is moved from the second position to the first position.

With this configuration, after the subcutaneous administration of the drug is complete and the movable portion is moved to the first position, the leading end of the needle member is covered by the cover member, and this covered state can be maintained. For example, even when the movable portion is moved to the second position in the state in which the needle member is covered by the cover member, this covered state is maintained, and thus it is possible to prevent reuse of the needle member.

In the above-mentioned drug administration device, at the first position before use, the leading end of the needle member can be covered by the cover member.

With this configuration, at the first position before use, the needle member is covered by the cover member before subcutaneous administration is performed, and thus it is possible to prevent the needle member from coming into contact with other members, a human body, and the like.

In the above-mentioned drug administration device, the cover member can be configured to be capable of being detached from the movable portion together with the needle member.

With this configuration, the needle member can be detached from the movable portion together with the cover member after subcutaneous administration of the drug is complete. Accordingly, it is possible to prevent reuse of the drug administration device and to separately collect the needle member. In addition, since the needle member is covered by the cover member, it is possible to prevent contact with the needle member and to prevent reuse of the needle member.

In the above-mentioned drug administration device, the thickness of the needle member is not particularly limited, but it can be set to a gauge of 30 (the outer diameter is 0.30 mm) to 35 (the outer diameter is 0.15 mm) in order to efficiently administer the drug.

In the above-mentioned drug administration device, the length of a portion of the needle member protruding from a surface of the drug administration device that is to be fixed to the skin can be set to 1 to 10 mm (3/64 to 25/64 inches). With this configuration, subcutaneous administration of the drug can be effectively performed on a wide range of patients from infants to elderly persons in consideration of individual differences between skin thicknesses.

The above-mentioned drug administration device can further include an accommodation portion configured to accommodate at least the movable portion when the movable portion is located at the second position.

With this configuration, the accommodation portion prevents the movable portion and the operation portion located at the second position from being caught on clothes or the like.

In the above-mentioned drug administration device, the needle member can be configured to be capable of being rotated around the axis of the needle member relative to the movable portion.

With this configuration, in a case where the needle member is provided with a needle hole in the side surface or the leading end of the needle member is obliquely cut, it is possible to change the position in the radial direction at which a liquid drug is discharged by rotating the needle member around the axis. Thus, it is also possible to adjust the flow rate of the liquid.

A first drug administration system according to the present invention includes: at least one of the above-described drug administration devices; at least one drug container; and a drug line for supplying a drug from the drug container to the drug administration device.

This first drug administration system is suitable for administering drugs such as an infusion in a large amount. That is, an infusion or the like can be stored in the drug container and administered to the drug administration device via the drug line.

A second drug administration system according to the present invention includes: at least one of the above-described drug administration devices; and a syringe capable of supplying a drug to the needle member.

This second drug administration system is suitable for administering drugs such as an insulin in a small amount. That is, insulin or the like, which is contained in the syringe, can be administered from the syringe to the drug administration device.

### EFFECT OF THE INVENTION

With the drug administration device and the drug administration system according to the present invention, subcutaneous administration of a liquid drug can be easily performed.

### BRIEF DESCRIPTION OF THE DRAWINGS

FIG. 1 is a perspective view of a drug administration device according to First Embodiment of the present invention.
FIG. 2 is a side view of FIG. 1.
FIG. 3 is a plan view of FIG. 1.
FIG. 4 is a cross-sectional view of FIG. 1.
FIG. 5A is a cross-sectional view of the drug administration device in a second position.
FIG. 5B is a perspective view of FIG. 5A.
FIG. 6 is a diagram illustrating examples of positions on the anterior side and the posterior side of a body at which the drug administration device shown in FIG. 1 is to be arranged.
FIG. 7 is a cross-sectional view illustrating a method of using the drug administration device shown in FIG. 1.
FIG. 8 is a cross-sectional view illustrating the method of using the drug administration device shown in FIG. 1.
FIG. 9 is a cross-sectional view illustrating a state in which an operation portion and a needle member are removed from the drug administration device shown in FIG. 1.
FIG. 10 is a perspective view of a drug administration device according to Second Embodiment of the present invention (first position).
FIG. 11 is a cross-sectional view of FIG. 10.
FIG. 12 is a cross-sectional view of the drug administration device in a second position.
FIG. 13 is a cross-sectional view of FIG. 12.
FIG. 14 is a cross-sectional view of the drug administration device illustrating a state in which the drug administration device is making a transition from a first position to a second position.
FIG. 15 is a cross-sectional view illustrating a method of using the drug administration device shown in FIG. 10.
FIG. 16 is a cross-sectional view illustrating a method of using the drug administration device shown in FIG. 10.
FIG. 17 is a cross-sectional view illustrating a state in which an operation portion and a needle member are removed from the drug administration device shown in FIG. 10.
FIG. 18 is a perspective view illustrating another example of the drug administration device shown in FIG. 1.
FIG. 19 is a plan view of FIG. 18.
FIG. 20 is a cross-sectional view of FIG. 18.
FIG. 21 is a cross-sectional view illustrating another example of the drug administration device shown in FIG. 1.
FIG. 22 is a cross-sectional view illustrating another example of the drug administration device shown in FIG. 1.
FIG. 23 is a cross-sectional view illustrating another example of the drug administration device shown in FIG. 1.
FIG. 24 is a cross-sectional view illustrating another example of the drug administration device shown in FIG. 1.
FIG. 25A is a perspective view illustrating another example of the drug administration device shown in FIG. 1.
FIG. 25B is a perspective view illustrating another example of the drug administration device shown in FIG. 1.
FIG. 25C is a perspective view illustrating another example of the drug administration device shown in FIG. 1.
FIG. 25D is a perspective view illustrating another example of the drug administration device shown in FIG. 1.
FIG. 25E is a cross-sectional view illustrating another example of the drug administration device shown in FIG. 1.
FIG. 26 is a cross-sectional view illustrating another example of the drug administration device shown in FIG. 1.
FIG. 27 is a side view illustrating another example of the drug administration device shown in FIG. 1.
FIG. 28 is a side view illustrating another example of the drug administration device shown in FIG. 1.
FIG. 29 is a side view illustrating another example of the drug administration device shown in FIG. 1.
FIG. 30 is a cross-sectional view illustrating another example of the drug administration device shown in FIG. 1.
FIG. 31 is a cross-sectional view illustrating another example of the drug administration device shown in FIG. 1.
FIG. 32 is a cross-sectional view illustrating a method of using the drug administration device shown in FIG. 31.
FIG. 33 is a cross-sectional view illustrating the method of using the drug administration device shown in FIG. 31.
FIG. 34 is a cross-sectional view illustrating the method of using the drug administration device shown in FIG. 31.
FIG. 35 is a cross-sectional view illustrating the method of using the drug administration device shown in FIG. 31.
FIG. 36 is a cross-sectional view illustrating another example of the drug administration device shown in FIG. 1.
FIG. 37 is a cross-sectional view illustrating a method of using the drug administration device shown in FIG. 36.
FIG. 38 is a cross-sectional view illustrating another example of the drug administration device shown in FIG. 1.
FIG. 39 is a cross-sectional view illustrating a method of using the drug administration device shown in FIG. 38.
FIG. 40 is a cross-sectional view illustrating the method of using the drug administration device shown in FIG. 38.
FIG. 41 is a plan view of a stopper.
FIG. 42 is a perspective view illustrating a method of using the stopper shown in FIG. 41.
FIG. 43 is a perspective view illustrating the method of using the stopper shown in FIG. 41.
FIG. 44 is a perspective view illustrating another example of the drug administration device shown in FIG. 1.
FIG. 45 is a perspective view illustrating a method of using the drug administration device shown in FIG. 44.
FIG. 46 is a cross-sectional view of FIG. 45.
FIG. 47 is a cross-sectional view illustrating another example of FIG. 45.
FIG. 48 is a cross-sectional view illustrating another example of the drug administration device shown in FIG. 1.
FIG. 49A is a perspective view illustrating another example of the drug administration device according to the present invention.
FIG. 49B is a perspective view illustrating another example of the drug administration device according to the present invention.
FIG. 50 is a perspective view illustrating another example of the drug administration device according to the present invention.
FIG. 51A is a side view illustrating another example of the drug administration device according to the present invention (first position).
FIG. 51B is a plan view of FIG. 51A.
FIG. 51C is a side view of FIG. 51A (second position).
FIG. 52A is a perspective view illustrating another example of the drug administration device according to the present invention (first position).
FIG. 52B is a perspective view illustrating another example of the drug administration device according to the present invention (second position).
FIG. 53A is a perspective view illustrating another example of the drug administration device according to the present invention (first position).
FIG. 53B is a perspective view illustrating another example of the drug administration device according to the present invention (second position).
FIG. 54A is a perspective view illustrating another example of the drug administration device according to the present invention (first position).
FIG. 54B is a perspective view illustrating another example of the drug administration device according to the present invention (second position).
FIG. 55A is a perspective view illustrating another example of the drug administration device according to the present invention (first position).
FIG. 55B is a front view of FIG. 55A.
FIG. 56A is a perspective view illustrating another example of the drug administration device according to the present invention (second position).
FIG. 56B is a front view of FIG. 56A.
FIG. 57A is a perspective view illustrating another example of the drug administration device according to the present invention (first position).
FIG. 57B is a perspective view illustrating another example of the drug administration device according to the present invention (second position).
FIG. 58A is a cross-sectional view illustrating another example of the drug administration device according to the present invention (first position).
FIG. 58B is a side view of FIG. 58A.
FIG. 59A is a cross-sectional view illustrating another example of the drug administration device according to the present invention. (second position).
FIG. 59B is a side view of FIG. 59A.
FIG. 60A is a cross-sectional view illustrating another example of the drug administration device according to the present invention (first position).
FIG. 60B is a cross-sectional view illustrating another example of the drug administration device according to the present invention (second position).
FIG. 61A is a cross-sectional view illustrating another example of the drug administration device according to the present invention (first position).
FIG. 61B is a cross-sectional view illustrating another example of the drug administration device according to the present invention (second position).
FIG. 62A is a cross-sectional view illustrating another example of the drug administration device according to the present invention (first position).
FIG. 62B is a cross-sectional view illustrating another example of the drug administration device according to the present invention (second position).
FIG. 63A is a perspective view illustrating another example of the drug administration device according to the present invention (first position).
FIG. 63B is a perspective view illustrating another example of the drug administration device according to the present invention (second position).
FIG. 64A is a cross-sectional view illustrating another example of the drug administration device according to the present invention (first position).
FIG. 64B is a cross-sectional view illustrating another example of the drug administration device according to the present invention (second position).
FIG. 65A is a perspective view illustrating another example of the drug administration device according to the present invention.
FIG. 65B is a perspective view illustrating another example of the drug administration device according to the present invention.
FIG. 66 is a cross-sectional view illustrating another example of the drug administration device according to the present invention.
FIG. 67 is a perspective view illustrating the lower side of another example of the drug administration device according to the present invention.
FIG. 68 is a cross-sectional view illustrating another example of the drug administration device according to the present invention.
FIG. 69 is a cross-sectional view illustrating another example of the drug administration device according to the present invention.
FIG. 70 is a perspective view illustrating another example of the drug administration device according to the present invention.
FIG. 71 is a cross-sectional view of FIG. 70.

### EMBODIMENTS OF THE INVENTION

### A. First Embodiment

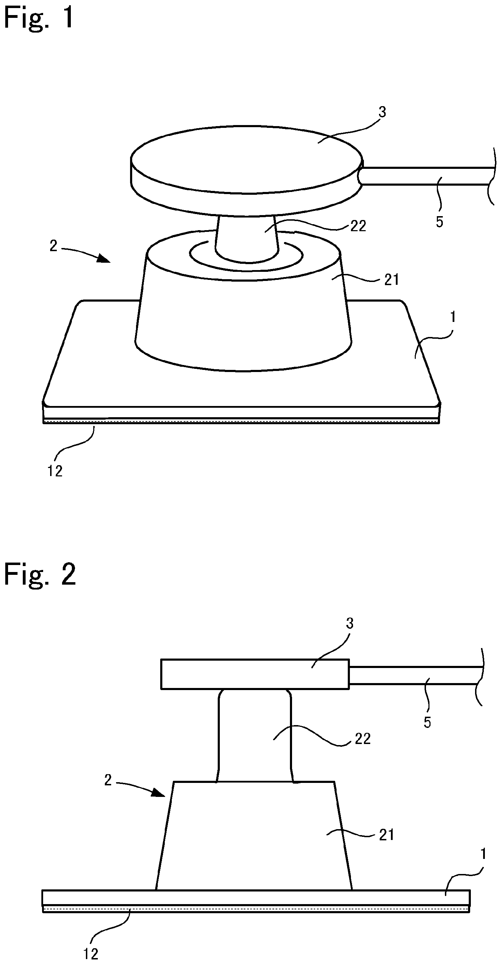

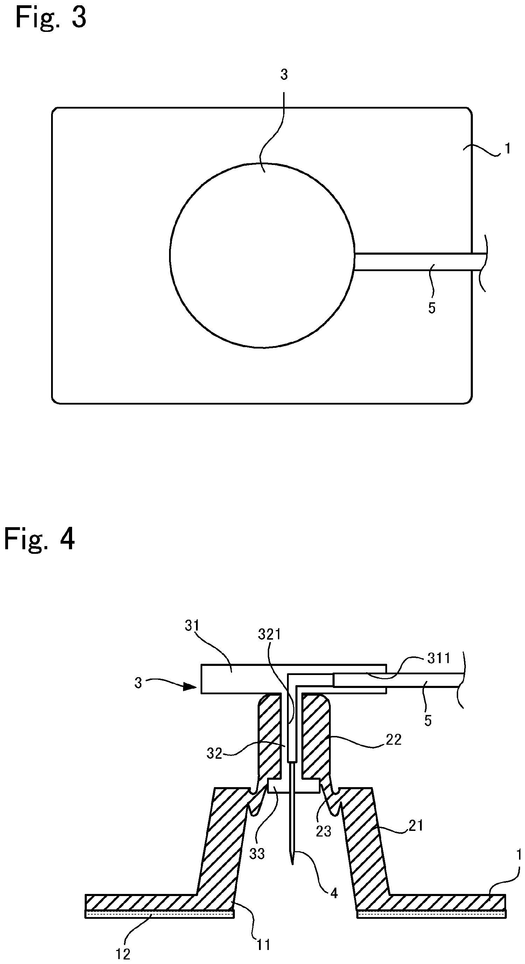

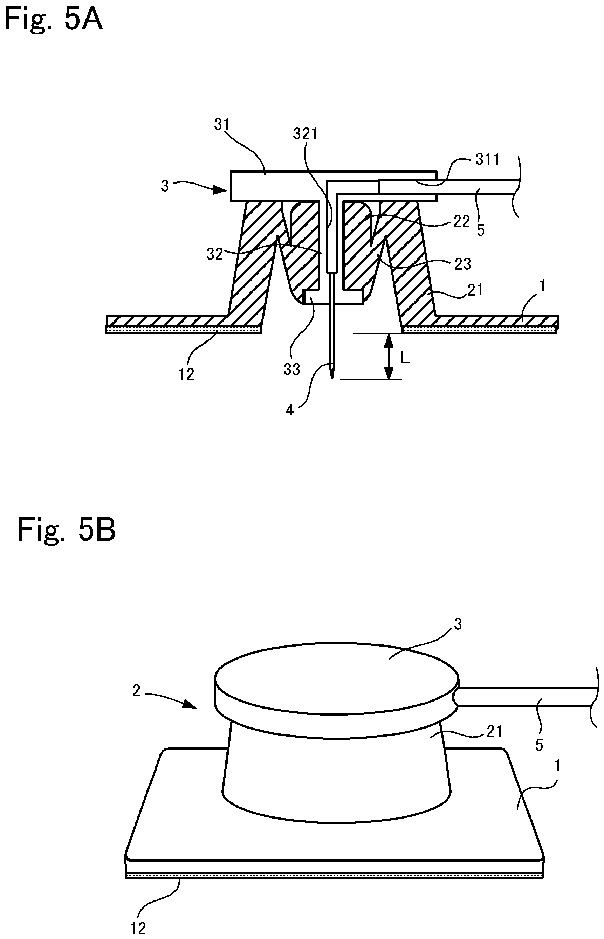

Hereinafter, First Embodiment of a drug administration device according to the present invention will be described with reference to the drawings. FIG. 1 is a perspective view of a drug administration device according to this embodiment (first position), FIG. 2 is a side view of FIG. 1, FIG. 3 is a plan view of FIG. 1, FIG. 4 is a cross-sectional view of FIG. 1 in an axial direction, FIG. 5A is a cross-sectional view of the drug administration device in a second position, and FIG. 5B is a perspective view of the drug administration device in the second position. All drug administration devices described in this specification, including First Embodiment below, are used to subcutaneously administer a liquid drug. Examples of the liquid drug include drugs to be administered in a large amount, such as an infusion (e.g., a physiological saline solution), and drugs to be administered in a small amount, such as antibacterial drugs and insulin. The administration route is not limited to subcutaneous administration, and the drug administration device can be used to intracutaneously administer a drug, or to administer a drug to a subcutaneous fat layer or a muscle layer, by adjusting the length of a needle of the drug administration device. This embodiment will be described in detail below.

### A-1. Overview of Drug Administration Device

As shown in FIGS. 1 to 5, the drug administration device according to this embodiment includes a base 1 that is formed in a rectangular plate shape, and a drug administration portion 2 that is integrally formed on the base 1.

The base 1 is provided with a circular through hole 11, and the drug administration portion 2 is provided covering the through hole 11. An adhering material 12 is applied to the lower surface of the base 1, and a release sheet (not shown) that covers the adhering material 12 is attached until the drug administration device is to be used. It should be noted that, when the base 1 is large in size, a release sheet can be divided into a plurality of pieces and then attached to the adhering material 12. A release sheet may be arranged covering the through hole 11 or attached to the adhering material excluding the portion at which the through hole 11 is provided. An adhering sheet or another seal material may be attached to the lower surface of the base 1 instead of applying the adhering material 12 to the lower surface of the base 1. Alternatively, the drug administration device can also be fixed to the skin by attaching a piece of tape such as adhering tape or surgical tape onto both the base 1 and the skin instead of providing the base 1 with the adhering material 12. In this case, the base 1 may be attached to the skin using a piece of tape after the base 1 is brought into contact with the skin, or a piece of tape may be attached to the base 1 in advance. It will be appreciated that the adhering material 12 and a piece of tape can also be used together.

The drug administration portion 2 includes a main body portion 21 with a cylindrical tubular shape that is attached to the base 1 and surrounds the through hole 11, a movable portion 22 with a cylindrical tubular shape that can be moved in an axial direction in the internal space of the main body portion 21, and a coupling portion 23 that couples the movable portion 22 and the main body portion 21 to each other, and these are formed of an elastic material, which will be described later, as a single body. It should be noted that the axial direction means a direction in which the axis of the cylindrical tubular shape formed by the main body portion 21 extends.

The coupling portion 23 is formed in an annular shape and couples the circumferential edge of the lower end of the movable portion 22 and the circumferential edge of the upper opening of the main body portion 21 to each other. Also, the coupling portion 23 is formed in a band shape having a predetermined width between the main body portion and the movable portion. Accordingly, when the coupling portion 23 extends upward from the circumferential edge portion (first end portion) of the upper opening of the main body portion 21 as shown in FIG. 4, the movable portion 22 is arranged at a position spaced apart from the base 1 (referred to as "first position" hereinafter), whereas, when the coupling portion 23 extends downward from the circumferential edge portion of the upper opening of the main body portion 21 as shown in FIGS. 5A and 5B, the movable portion 22 is arranged at a position near the base 1 (referred to as "second position" hereinafter). It should be noted that, at the second position, the lower end of the movable portion 22 may enter the through hole 11.

When the movable portion 22 is moved between the two positions, the coupling portion 23 is elastically deformed. Therefore, the movable portion 22 is held immovably at each of the two positions as long as an external force is not applied thereto. On the other hand, when an external force is applied, the movable portion 22 is moved between the two positions. In this embodiment, as shown in FIG. 5A, when the movable portion 22 is located at the second position, the coupling portion 23 extends from the upper opening of the main body portion 21 toward the lower end portion of the movable portion 22 without the application of a load (stable state). On the other hand, as shown in FIG. 4, when the movable portion 22 is located at the first position, the coupling portion 23 bends and extends upward (semistable state). That is, an elastic load is applied to the coupling portion 23 located at the first position. Accordingly, when a downward force is applied to the movable portion 22 located at the first position, the coupling portion 23 is elastically deformed to return to the stable state in which the coupling portion 23 does not bend, and is moved to the second position. Therefore, the movable portion 22 is quickly moved to the second position due to an elastic force. The movable portion 22 is provided with an internal space that extends in the vertical direction and whose upper and lower ends are open to the outside, and a fixation portion 32 of an operation portion 3, which will be described later, is inserted into this internal space.

In this embodiment, the base 1, the main body portion 21, the coupling portion 23, and the movable portion 22 are formed of an elastic material as a single body, and examples of the elastic material include elastomers such as silicone rubber, natural rubber, synthetic natural rubber, butadiene rubber, chloroprene rubber, and urethane rubber, and metals. For example, these portions can be formed as a single body through injection molding.

The drug administration device is further provided with the operation portion 3, and a needle member 4 attached to the operation portion 3. A channel extending in the axial direction is provided inside the needle member 4. The operation portion 3 includes a disk-shaped basal portion 31 whose external size is larger than the external size of the main body portion 21, and a cylindrical fixation portion 32 that protrudes from the center of the lower surface of the basal portion 31, and these are formed as a single body. A flange portion 33 that protrudes outward in the radial direction is formed at the leading end of the fixation portion 32. Furthermore, a linear first passage 311 is formed extending from the outer circumferential edge of the basal portion 31 to the center thereof, and a linear second passage 321 is formed extending from the vicinity of the upper end of the fixation portion 32 to the vicinity of the center thereof in the axial direction. An end portion of the first passage 311 near the center of the basal portion 31 is in communication with the upper end of the second passage 321, and a passage with an L shape overall is formed inside the operation portion 3. A drug line 5 including a tube, a spike, a drip chamber, a roller clamp, a connector, and the like is coupled to an end portion of the first passage 311 near the outer circumferential edge of the basal portion 31 via a connector (not shown: for connecting the drug line and the operation portion) in order to supply a drug. It should be noted that the drug line 5 may be detachably or undetachably fixed to the operation portion 3 via a connector. As described later, the connector may also be provided via a tube provided to the operation portion 3, for example, instead of being directly provided to the operation portion 3.

The above-described needle member 4 is inserted into the lower end of the fixation portion 32 and is thus fixed, and extends to the second passage 321. Therefore, a drug supplied to the first passage 311 is discharged through a hole provided in the needle member 4 via the second passage 321. The needle member 4 can be configured to have a thickness of a gauge of 30 (the outer diameter is 0.30 mm) to 35 (the outer diameter is 0.15 mm), for example. In addition, the hole provided in the needle member 4 encompasses a hole provided at the tip of the needle and a hole (side hole) provided in a side portion of the needle, and there is no particular limitation on the position of the hole. Furthermore, a plurality of holes may also be provided.

It should be noted that various materials can be used to form the operation portion 3. The operation portion 3 can be formed using the same elastic material as the elastic material used to form the drug administration portion 2, or a resin material such as hard resin (e.g., polypropylene, polyethylene terephthalate, or polyethylene). As the manufacturing method, the needle member 4 can be fixed to the operation portion 3 by performing insert molding on the needle member 4 together with the operation portion 3, or using an adhesion means such as an adhesive. It should be noted that the needle member 4 can encompass a member constituted by only a needle as well as a member constituted by a needle and a needle base. Accordingly, it is also possible to arrange a needle base to which a needle is attached in a mold and then perform insert molding.

The thus-formed operation portion 3 can be fixed by inserting the fixation portion 32 into the internal space of the movable portion 22. At this time, the flange portion 33 of the fixation portion 32 protrudes outward from the lower end of the movable portion 22 in the radial direction and thus serves as a dislodgement prevention portion, and therefore, the operation portion 3 is fixed to the movable portion 22. When the operation portion 3 is fixed to the movable portion 22, the needle member 4 is accommodated in the main body portion 21 when the movable portion 22 is located at the first position as shown in FIG. 4, whereas the needle member 4 protrudes from the through hole 11 of the base 1 in the case where the movable portion 22 is located at the second position, as shown in FIG. 5A. At this time, a length L of a portion of the needle member 4 protruding from the lower surface of the adhering material 12 can be set to 1 to 10 mm (3/64 to 25/64 inches), for example, for the purpose of subcutaneous administration. It should be noted that the subcutaneous administration device of this embodiment is provided with only a single needle member, but a plurality of needle members can also be provided.

It should be noted that there is no particular limitation on the method of fixing the fixation portion 32 to the movable portion 22. For example, a fixation portion 32 provided with no flange portion 33 can be pressed into the movable portion 22 and fixed with a frictional force, or the fixation portion 32 can be fixed to the movable portion 22 using an adhesive, or the fixation portion 32 can be fixed to the movable portion 22 through thermal welding. In the drug administration device in the initial state, the movable portion 22 is located at the first position. The drug administration device is packed and sterilized in this state, and then shipped.

### A-2. Method for Using Drug Administration Device

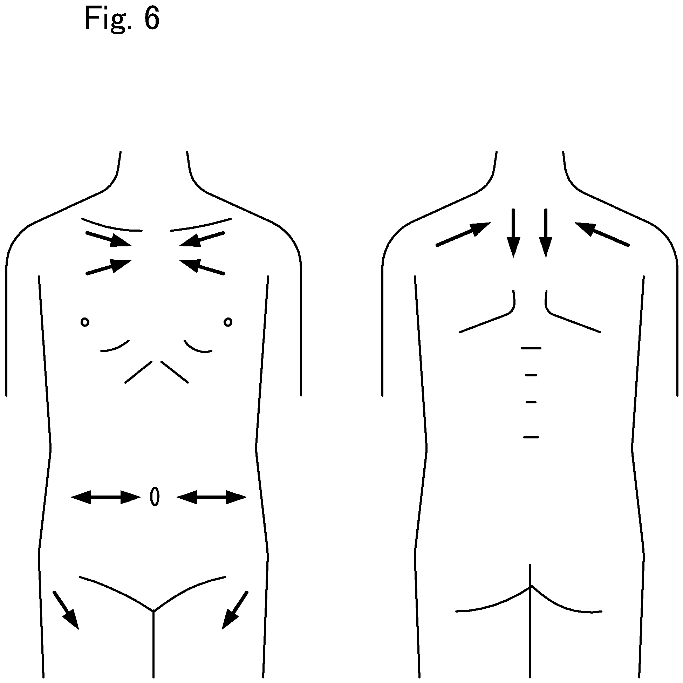

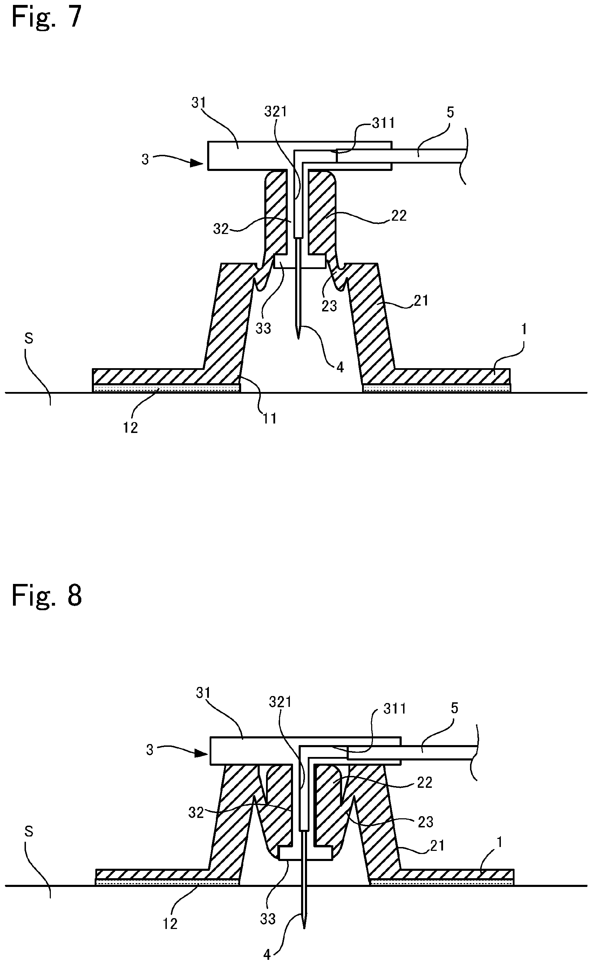

Next, a method for using the drug administration device having the above-mentioned configuration will be described. As mentioned above, the movable portion 22 is located at the first position in the initial state. Then, the release sheet is removed from the base 1 and the adhering material 12 is exposed. Next, an infusion bag (not shown) containing a drug is connected to the drug line 5. Thus, the drug discharged from the infusion bag is supplied to the needle member 4 via the drug line 5. As shown in FIG. 7, once the drug line 5 and the needle member 4 are filled with the drug, the adhering material 12 is attached to the surface of skin S to be subjected to subcutaneous administration. It should be noted that, as the portion at which the attachment of this device and puncture are to be performed, a portion with a large body surface area and sufficient subcutaneous room, such as the portions indicated by arrows in FIG. 6, is recommended, for example. Subsequently, the operation portion 3 is pressed toward the base 1, and thus the movable portion 22 is moved to the second position. Thus, as shown in FIG. 8, the needle member 4 passes through the through hole 11 and is quickly inserted into the skin S, and the drug is subcutaneously administered from the needle member 4. It should be noted that the drug line 5 and the infusion bag may be coupled to each other after the base 1 is attached to the skin.

After that, when a predetermined period of time has elapsed and the administration of the drug is complete, fingers are used to hold the operation portion 3 and pull it up. Thus, as shown in FIG. 7, the movable portion 22 is moved to the first position, and the needle member 4 is removed from the skin S. Subsequently, the drug line 5 is removed, the base 1 is removed from the skin, and the drug administration device is discarded as needed. Here, the force required to move the movable portion 22 to the second position is smaller than the force required to move the movable portion 22 to the first position, thus making it possible to realize a stable puncture operation and a stable needle removal operation.

### A-3. Features of the drug administration device

As described above, with this embodiment, moving the movable portion 22 from the first position to the second position makes it possible to cause the leading end portion of the needle member 4 to protrude from the through hole 11 of the base 1 and insert the needle member 4 into the skin. Since the drug supplied through the drug line 5 can be discharged from the leading end portion of the needle member 4, the drug can be subcutaneously administered merely by moving the movable portion 22 to the second position. Accordingly, a patient or user can perform subcutaneous administration of the drug with a simple operation. In particular, the drug can be administered by performing operations such as pushing down the operation portion 3 and pulling the operation portion 3 up, and the needle member 4 is accommodated in the main body portion 21, thus making it possible to reduce a psychological barrier (e.g., inserting and removing a needle) felt by a user such as a patient. In addition, the base 1, the main body portion 21, the movable portion 22, and the coupling portion 23 of this drug administration device are formed of an elastic material as a single body, thus making it possible to simplify the structure thereof.

At the first position before use, the needle member 4 is accommodated in the main body portion 21, and the leading end of the needle member 4 is located higher than the base 1, thus making it possible to prevent the needle member 4 from being inserted into a patient by mistake before use. Accordingly, the drug administration device is very safe and hygienic.

The base 1 to be arranged on the skin is also formed of an elastic material and can thus be deformed so as to follow the skin surface and arranged thereon. In addition, the base 1 is formed as a single body together with the main body portion 21, the movable portion 22, and the coupling portion 23, and therefore, the main body portion 21, the movable portion 22, and the coupling portion 23 can also follow the deformation of the base 1 following the skin. Accordingly, the needle member 4 protruding from the movable portion 22 can precisely protrude from the through hole 11 of the base 1, and even when the drug administration device is attached to the skin for a long period of time, a risk that the drug administration device will come off from the skin can be reduced.

Moreover, the adhering material 12 is applied on the base 1, thus making it possible to firmly hold the drug administration device on the skin and to reliably prevent the device from coming off from the skin during administration of the drug over a long period of time.

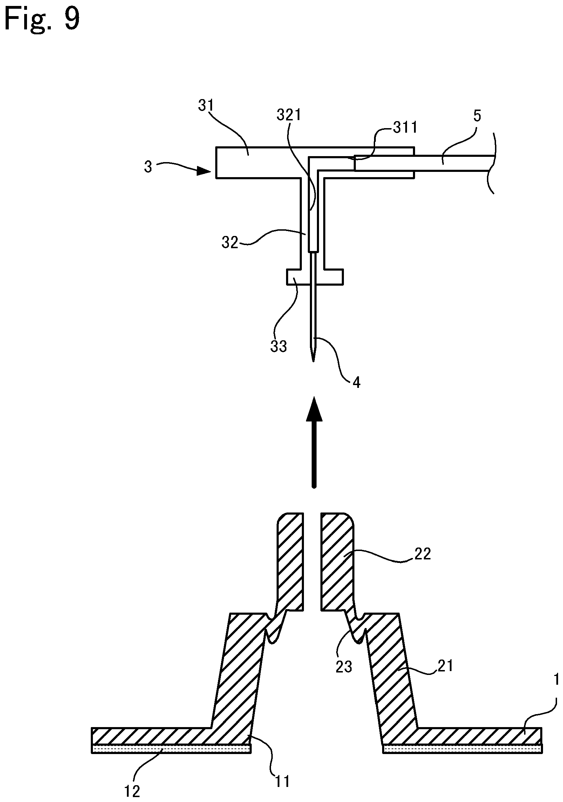

The needle member 4 and the operation portion 3 are formed as a single body, thus making it possible to remove them from the movable portion 22 and discard them. Therefore, it is possible to prevent reuse of the drug administration device. As shown in FIG. 9, separately collecting the components excluding the needle member 4 and the operation portion 3, namely the base 1, the main body portion 21, the coupling portion 23, and the movable portion 22, which are formed as a single body, makes it possible to reuse these members. Accordingly, it is possible to reduce the total waste amount.

### B. Second Embodiment

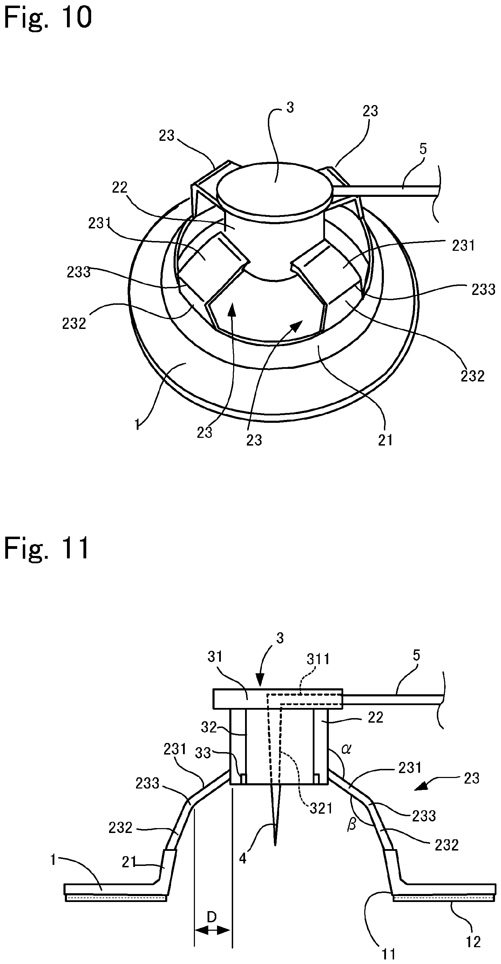

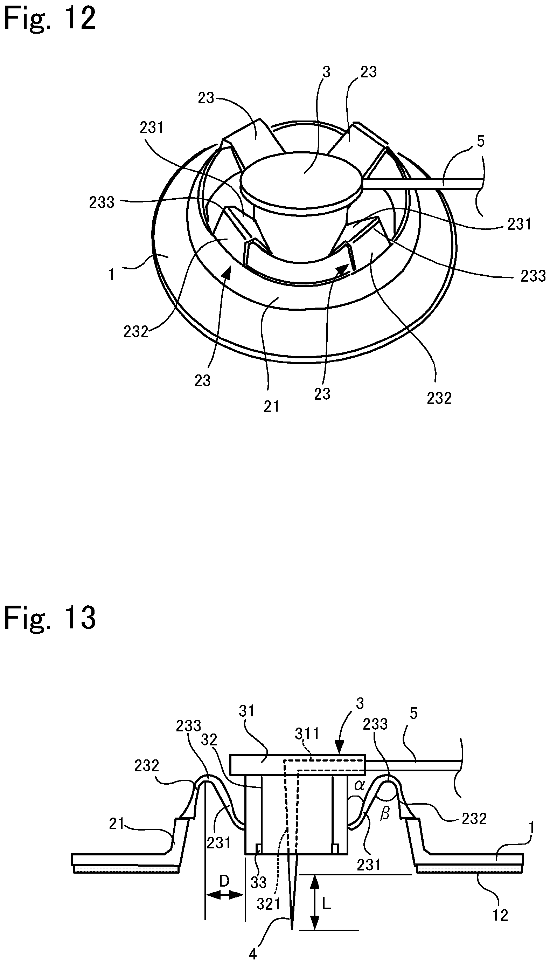

Hereinafter, Second Embodiment of a drug administration device according to the present invention will be described with reference to the drawings. The drug administration device according to this embodiment mainly differs from First Embodiment in the configuration of the coupling portion. The other configurations are substantially the same, and therefore, the same configurations are denoted by the same reference numerals, and descriptions thereof are omitted. FIG. 10 is a perspective view of a drug administration device according to this embodiment (first position), FIG. 11 is a cross-sectional view of FIG. 10, FIG. 12 is a perspective view of the drug administration device in a second position, and FIG. 13 is a cross-sectional view of FIG. 12. This embodiment will be described in detail below.

### B-1. Overview of Drug Administration Device

As shown in FIGS. 10 to 13, the drug administration device according to this embodiment includes a base 1, a main body portion 21, a movable portion 22, and a plurality of (four, in this embodiment) coupling portions 23 that couple the movable portion 22 and the main body portion 21 to each other, and these are formed of a resin material, which will be described later, as a single body. Hereinafter, the coupling portion will be mainly described.

The coupling portions 23 are formed in a plate shape, and are radially arranged at regular intervals, coupling the circumferential edge portion of the lower end portion of the movable portion 22 and the circumferential edge portion of the upper opening of the main body portion 21 to each other. Accordingly, a gap is formed between the adjacent coupling portions 23. The following is a more detailed description. Each of the coupling portions 23 includes a first portion 231 and a second portion 232, and the first portion 231 and the second portion 232 are bendably coupled to each other via a thin bendable portion 233. Moreover, the upper end portion of the first portion 231 and the lower end portion of the movable portion 22 are bendably coupled to each other. Accordingly, the coupling portion 23 is bent at two positions including the position at which the coupling portion 23 and the movable portion 22 are coupled to each other. Thus, the movable portion 22 can be moved between a first position shown in FIGS. 10 and 11 and a second position shown in FIGS. 12 and 13. It should be noted that the configuration of the bendable portion 233 is not limited to that obtained by thinning a portion of the coupling portion 23, and the bendable portion 233 can be formed using means for providing a cutout or providing a crease during manufacturing.

For example, in the first position shown in FIGS. 10 and 11, each of the first portions 231 of the coupling portions 23 and the side surface of the movable portion 22 (a portion of the side surface located above the coupling portion) are coupled to each other such that an angle α formed therebetween is an obtuse angle. In addition, each of the coupling portions 23 is bent such that an angle β formed at the bendable portion 233 by the first portion 231 and the second portion 232 is an obtuse angle (first state). Thus, the movable portion 22 is supported above the main body portion 21. At this time, the leading end of the needle member 4 is located higher than the lower surface of the base 1. That is, as described later, a state in which the needle member 4 is not inserted into the skin is maintained.

On the other hand, in the second position shown in FIGS. 12 and 13, each of the first portions 231 of the coupling portions 23 and the side surface of the movable portion 22 are coupled to each other such that the angle α is an acute angle. In addition, each of the coupling portions 23 is bent such that the angle β formed at the bendable portion 233 is an acute angle (second state). Thus, the movable portion 22 is located below the first position, and at least a portion thereof enters the main body portion 21. Thus, the leading end of the needle member 4 is located below the lower surface of the base 1. That is, as described later, a state in which the needle member 4 is inserted into the skin is maintained.

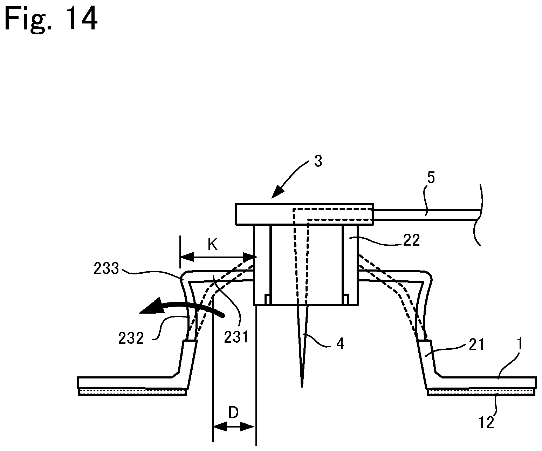

FIG. 14 is a cross-sectional view of the drug administration device illustrating a state in which the drug administration device is making a transition from the first position to the second position. As shown in FIG. 14, at the first position, a distance D in the horizontal direction between the outer circumferential surface of the movable portion 22 and the upper end of the second portion 232 of the coupling portion 23 is shorter than a length K of the first portion 231. Therefore, when the movable portion 22 is moved from the first position to the second position, the coupling portions 23 are bent at an acute angle while the second portions 232 of the coupling portions 23 are elastically deformed to extend outward relative to the main body portion 21 in the radial direction. When the movable portion 22 is moved from the second position to the first position, the second portions 232 of the coupling portions 23 are also elastically deformed to extend outward relative to the main body portion 21 in the radial direction. Therefore, when the movable portion 22 is moved between the first position and the second position, it is necessary to apply an external force such that the second portions 232 are elastically deformed, and thus the movable portion 22 is held immovably in the state in which an external force is not applied thereto, namely the state in which the movable portion is located at the first position and the second position. In other words, the movable portion 22 is held immovably at these positions as long as an external force is not applied thereto and the movable portion 22 is not elastically deformed. Moreover, for example, setting the force required to move the movable portion 22 to the second position to be smaller than the force required to move the movable portion 22 to the first position makes it possible to realize a stable puncture operation and a stable needle removal operation as described later.

In particular, when the movable portion 22 located at the first position is pressed from above, the coupling portions 23 are bent while the second portions 232 are elastically deformed, and thus the movable portion 22 is quickly moved to the second position. At this time, the second portions 232 of the coupling portions 23 are elastically deformed in a similar manner to extend outward in the radial direction, and therefore, the movable portion 22 and the needle member 4 is moved straight parallel to the vertical axis, and as a result, the needle member 4 is inserted into the skin at a right angle.

The movable portion 22 is provided with an internal space that extends in the vertical direction and whose upper and lower ends are open to the outside, and a fixation portion 32 of an operation portion 3, which will be described later, is to be inserted into this internal space.

In this embodiment, the base 1, the main body portion 21, the coupling portions 23, and the movable portion 22 are formed of a material that can be elastically deformed as a single body, and examples of such a material include the materials described in First Embodiment above as well as hard resins such as polyolefins (e.g., polypropylene and polyethylene), polyesters (e.g., polyethylene terephthalate), and ABS, and metals. In particular, the above-described hard resins (e.g., polyolefins, polyesters, and ABS) are preferable. Moreover, hard plastic defined by JIS K6900 (plastic having a bending elastic modulus or tensile elastic modulus of 700 MPa or greater) can be used, and polypropylene, ABS, and the like correspond to thus-defined hard plastic. These portions can be molded as a single body through injection molding, for example.

On the other hand, a configuration can also be employed in which one or more of the base 1, the main body portion 21, the coupling portions 23, and the movable portion 22 are formed of a different material. Moreover, a configuration can also be employed in which the bendable portions 233 of the coupling portions 23 are formed using separate materials such as known hinges, or in which each of the coupling portions 23 is formed as a movable member by joining the first portion 231 and the second portion 232 via a highly flexible member.

The drug administration device further includes the operation portion 3 and a needle member 4 attached to the operation portion 3. These configurations are the same as those of First Embodiment above, and descriptions thereof are omitted.

### B-2. Method for Using Drug Administration Device

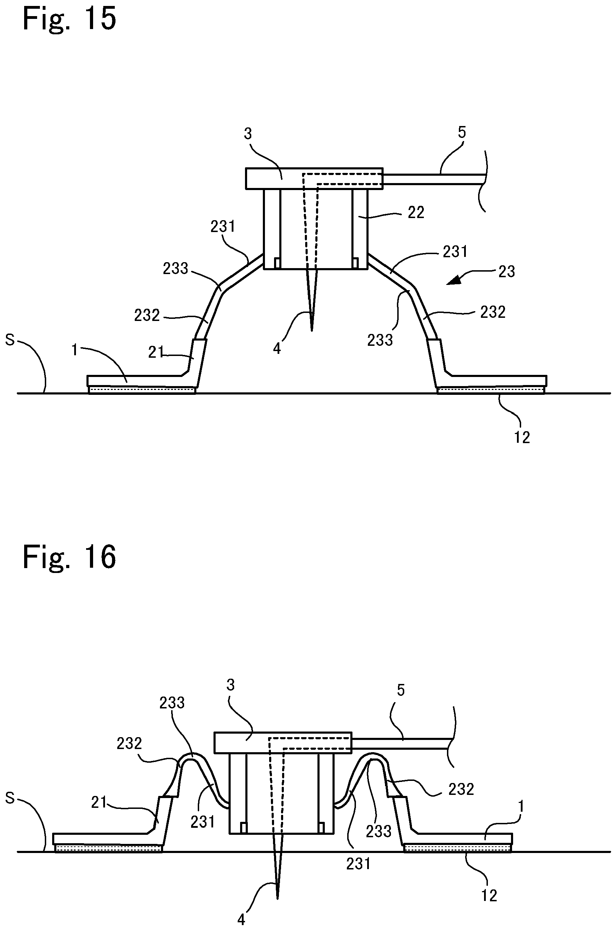

Next, a method for using the drug administration device having the above-mentioned configuration will be described. This method is substantially the same as that of First Embodiment. As mentioned above, the movable portion 22 is located at the first position in the initial state. Then, the release sheet is removed from the base 1 and the adhering material 12 is exposed. Next, an infusion bag (not shown) containing a drug is connected to the drug line 5. Thus, the drug discharged from the infusion bag is supplied to the needle member 4 via the drug line 5. As shown in FIG. 15, once the drug line 5 and the needle member 4 are filled with the drug, the adhering material 12 is attached to the surface of skin S to be subjected to subcutaneous administration. It should be noted that, the portion at which the attachment of this device and puncture are to be performed can be set to be the same as the above-described portions shown in FIG. 6. Subsequently, the operation portion 3 is pressed toward the base 1, and thus the movable portion 22 is moved to the second position. Thus, as shown in FIG. 16, the needle member 4 passes through the through hole 11 and is quickly inserted into the skin S, and the drug is subcutaneously administered from the needle member 4. It should be noted that the drug line 5 and the infusion bag may be coupled to each other after the base 1 is attached to the skin.

After that, when a predetermined period of time has elapsed and the administration of the drug is complete, fingers are used to hold the operation portion 3 and pull it up. Thus, as shown in FIG. 15, the movable portion 22 is moved to the first position, and the needle member 4 is removed from the skin S. Subsequently, the drug line 5 is removed, the base 1 is removed from the skin, and the drug administration device is discarded as needed.

### B-3. Features of the drug administration device

As described above, with this embodiment, in the case where the drug administration device is formed of a resin material such as hard resin (e.g., polypropylene), for example, the movable portion 22 is firmly supported by the coupling portions 23, and thus the movable portion 22 can be stably dislocated. As a result, the needle tip can be moved from the first position to the second position along a stable trajectory, and thus it is possible to suppress oblique insertion of the needle member 4 into the skin. As described above, in order to elastically deform a resin material such as hard resin, this embodiment has a configuration in which a plurality of bendable coupling portions 23 are provided between the movable portion 22 and the main body portion 21 with intervals therebetween, and the coupling portions 23 are formed so as to be capable of being deformed to an extent that a user can operate them manually. Since the coupling portions 23 are radially arranged with predetermined intervals, the coupling portions 23 are equally deformed when the movable portion 22 is moved and can thus support the movable portion 22 in a balanced manner in the circumferential direction.

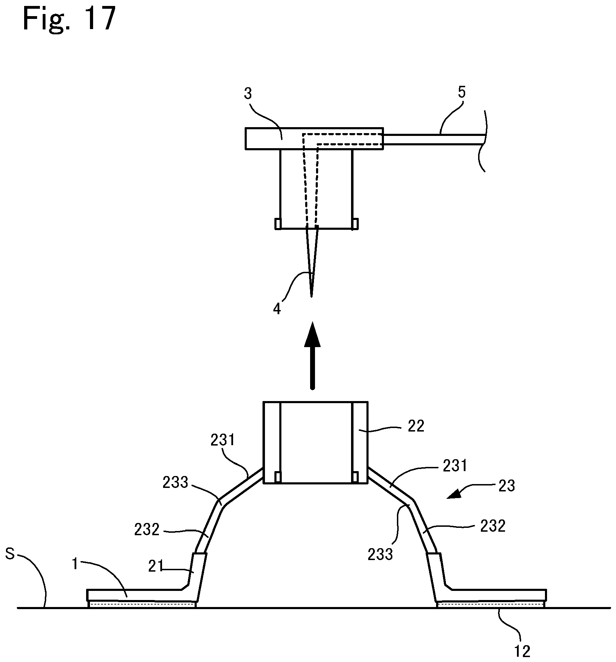

The needle member 4 and the operation portion 3 are formed as a single body, thus making it possible to remove them from the movable portion 22 as shown in FIG. 17 and discard them. This configuration is the same as that in First Embodiment above.

The number of coupling portions 23 is not limited to four, and it is sufficient that a plurality of coupling portions 23 are arranged. In particular, when the number of coupling portions 23 is increased, the movable portion 22 can be stably supported, but if the number of coupling portions 23 is excessively increased, it becomes difficult to deform all the coupling portions. Accordingly, it is preferable that the number of coupling portions is two to ten.

There is no particular limitation on the configuration of the coupling portion 23, and the coupling portion 23 may also be configured to be capable of being bent at a plurality of positions. However, a configuration is preferable in which the coupling portions 23 are elastically deformed while being moved between the first position and the second position. With this configuration, the movable portion 22 can be held at the first position and the second position as long as an external force is not applied thereto and the movable portion 22 is not elastically deformed.

Alternatively, the movable portion 22 may be configured such that it is necessary to apply an external force in order to hold the movable portion 22 at one of the positions. For example, the movable portion 22 can be forcibly held at one of the positions using a jig or the like.

There is no particular limitation on the positions at which the coupling portions 23 are provided. There is no particular limitation on the coupling positions of the coupling portions 23 as long as the movable portion 22 and the main body portion 21 are coupled to each other such that the movable portion 22 can be moved between the first position and the second position.

### C. Modified Examples

Although the embodiments of the present invention have been described above, the present invention is not limited to the above embodiments, and various modifications can be carried out without departing from the gist of the invention. For example, the following modifications can be carried out, and modified examples below can be implemented in combination as appropriate. Also, the above-described configurations of the embodiments and the configurations of the modified examples below can be implemented in combination as appropriate.

### C-1

Although the operation portion 3 is provided with the passages 311 and 321, and the needle member 4 is inserted into the fixation portion 32 of the operation portion 3 and thus fixed in the above-mentioned embodiment, there is no particular limitation on the configurations of the needle member 4 and the passages 311 and 321 for supplying a drug to the needle member 4, and various aspects are possible.

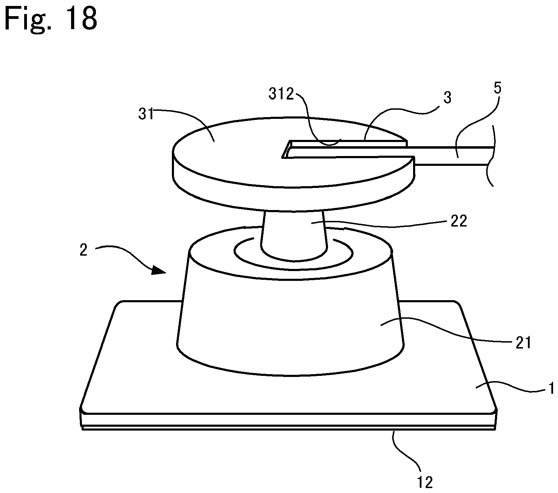

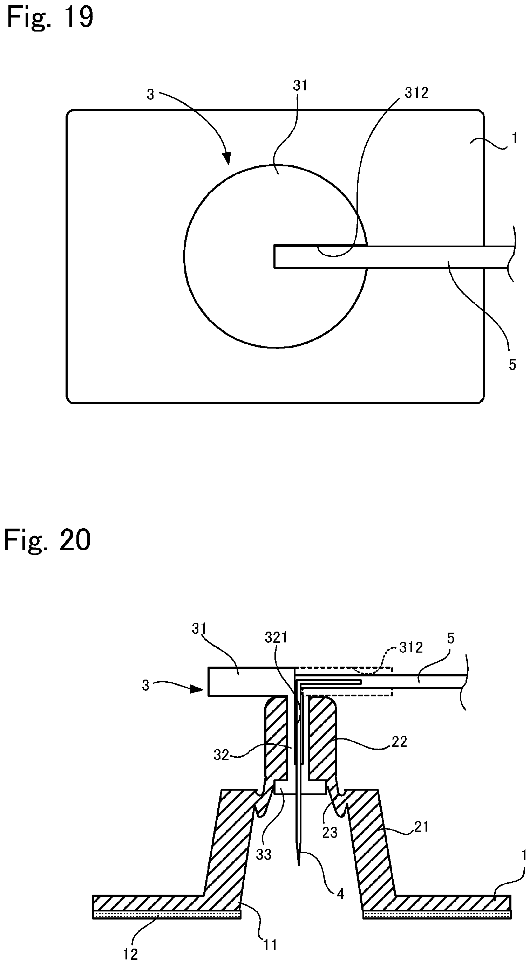

For example, as shown in FIGS. 18 to 20, a configuration can be employed in which the basal portion 31 of the operation portion 3 is provided with a cutout 312 at a portion corresponding to the above-described first passage 311, and this cutout 312 is provided with a connector (not shown), and a connector on the drug line 5 side (not shown) is inserted into that connector. At this time, the needle member 4 is formed in an L shape and can be configured such that one end portion thereof passes through the fixation portion 32 and the other end is inserted into the cutout 312 in the radial direction. Then, the other end portion of the needle member 4 is inserted into the drug line 5 that is inserted into the cutout 312. It should be noted that the needle member 4 can be fixed to the operation portion 3 by performing insert molding thereon together with the operation portion 3 and the connector.

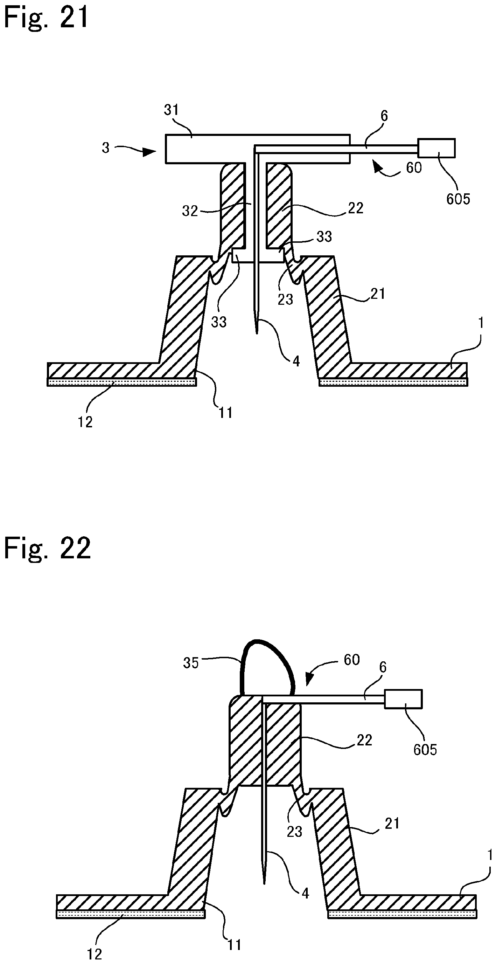

Alternatively, as shown in FIG. 21, an overall L-shaped needle module 60 is formed by coupling a tube 6 to the upper end portion of a linear needle member 4 at a right angle. At this time, the passage of the needle member 4 and the tube 6 are in communication with each other. The length of the tube 6 is set to be longer than the radius of the basal portion 31 of the operation portion 3. This needle module 60 is arranged in a mold, and then insert molding is performed to form the operation portion 3. This makes it possible to form the needle module 60 and the operation portion 3 as a single body. The tube 6 of the needle module 60 protrudes from the outer edge of the basal portion 31 of the operation portion 3, and thus the drug line 5 can be connected to a connector 605 provided at the end portion of the tube 6. It should be noted that a configuration may also be employed in which a channel such as that shown in FIG. 4 is formed inside the operation portion 3, and the tube 6, the channel, and the needle member 4 are in communication.

As described above, there is no particular limitation on the configuration for supplying a drug to the needle member 4. As shown in FIG. 4 described above, a configuration can also be employed in which the drug line 5 is attached to the operation portion 3, and an infusion bag is connected to the drug line 5. Moreover, as shown in FIG. 21, a configuration can also be employed in which the tube 6 is attached to the operation portion 3 or the needle member 4, and the drug line connected to an infusion bag is connected to the tube 6 via a connector or the like. Alternatively, a tubing member connected to an infusion bag can also be directly coupled to the needle member 4. Accordingly, in the present invention, a member that couples the needle member 4, the movable portion 22, or the operation portion 3 to a liquid drug supply source such as an infusion bag and supplies a liquid drug corresponds to the drug line. Such a drug line can also be configured by coupling a plurality of tubing members.

### C-2

Although the above-mentioned embodiment is configured such that the operation portion 3 whose external size is larger than that of the main body portion 21 is coupled to the movable portion 22, and fingers are used to hold the operation portion 3 and move the movable portion 22 from the second position to the first position, a configuration for facilitating movement of the movable portion 22 is not limited to this configuration. For example, the basal portion 31 of the operation portion may also be attached to a portion other than the upper end of the movable portion 22, and can also be formed in a flange shape that protrudes outward from the outer circumferential surface of the movable portion 22 in the radial direction (not shown). Moreover, as shown in FIG. 22, a thread member 35, which is an aspect of the operation portion, can also be attached to the movable portion 22. The thread member 35 can be formed as a single body with the movable portion 22 and fixed thereto through insert molding, for example. Moreover, the same material as that of the base 1, the main body portion 21, and the movable portion 22 can be used to form the thread member 35 as a single body therewith. As described above, fixing the two ends of the thread member 35 to the movable portion 22 makes it possible to easily move the movable portion 22 from the second position to the first position by pulling this thread member 35.

### C-3

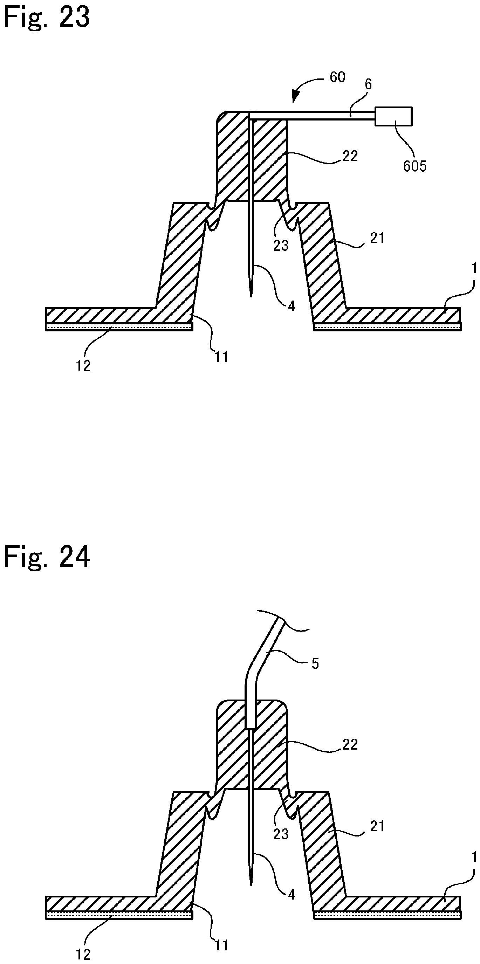

Although the needle member 4 and the operation portion 3 are formed as a single body and thus can be removed from the movable portion 22 by pulling them up in the above-mentioned embodiment, the operation portion 3 is not necessarily required. For example, as shown in FIG. 23, a configuration can also be employed in which the operation portion 3 is not provided, and the above-described needle module 60 is fixed to the movable portion 22 through injection molding or the like. Alternatively, as shown in FIG. 24, a configuration can also be employed in which a connector (not shown) is provided at the upper end portion of the needle member 4, and the drug line 5 is coupled thereto via this connector. In the aspects shown in FIGS. 23 and 24, the movable portion 22 can be moved between the positions by using fingers to directly hold and push the movable portion 22. Accordingly, there is also no particular limitation on the configuration of the movable portion 22, and it is sufficient the movable portion 22 is configured such that the needle member 4 can be attached thereto.

### C-4

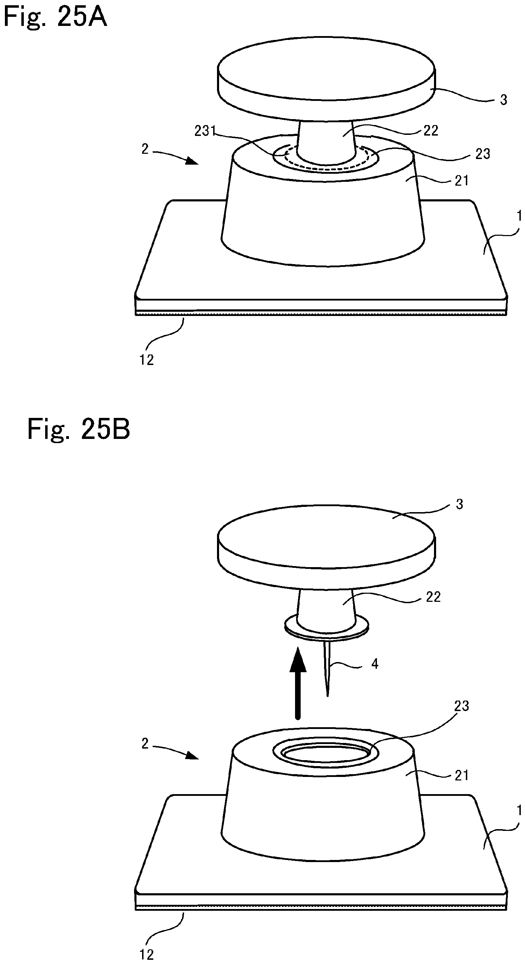

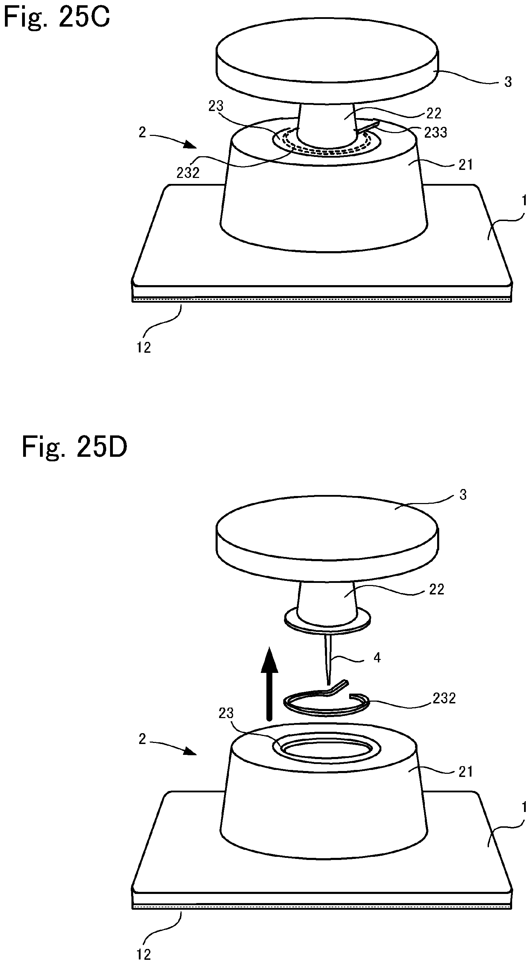

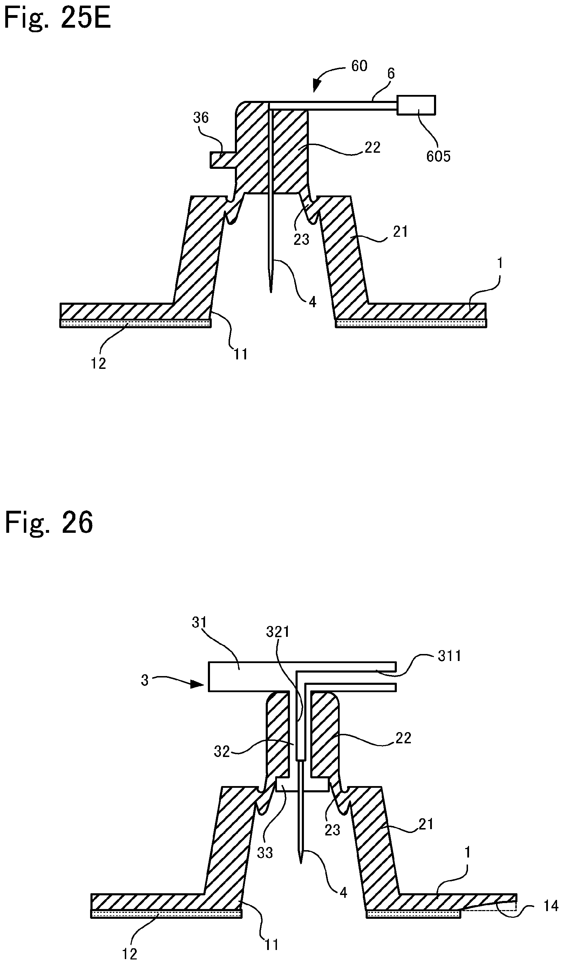

In order to separately collect the operation portion 3 including the needle module 60 or to prevent reuse of the drug administration device of the present invention after a drug has been administered using the drug administration device and the device has been removed from the skin, a configuration can be employed in which the operation portion 3 is removed from the movable portion 22 by strongly pulling the operation portion 3, for example. Moreover, as shown in FIG. 25A, a configuration can also be employed in which the coupling portion 23 is provided with an annular perforation portion 231, and thus the operation portion 3 including the needle module 60 can be torn off together with the movable portion 22. With this configuration, as shown in FIG. 25B, the coupling portion 23 is separated, and the movable portion 22 is detached from the main body portion 21 together with the needle member 4. Alternatively, as shown in FIG. 25C, a configuration can be employed in which the coupling portion 23 is provided with an annular breakage portion 232 constituted by perforations, and the breakage portion 232 is detached from the coupling portion 23 by pulling an end portion 233 of the breakage portion 232 protruding from the coupling portion 23. With this configuration, as shown in FIG. 25D, the coupling portion 23 is separated, and thus the movable portion 22 can be detached from the main body portion 21. Alternatively, as shown in FIG. 25E, a configuration can also be employed in which the movable portion 22 or the coupling portion 23 is provided with a grip 36, and this grip 36 is pulled to tear off the operation portion 3 including the needle module 60 together with the movable portion 22. It should be noted that the above-mentioned perforation portion or grip 36 forms the detaching means of the present invention.

### C-5

In order to facilitate removal of the base 1 from the skin, a configuration can also be employed in which the base 1 is provided with a recessed portion 14 on the lower surface as shown in FIG. 26 such that a gap is formed between the base 1 and the skin, for example. With this configuration, a finger can be inserted into the gap at which the recessed portion 14 is provided, and the finger can be used to hold the base 1 and remove it from the skin. Moreover, a configuration can also be employed in which no adhering material 12 is applied to the recessed portion 14 such that a finger can be more easily inserted into the recessed portion 14. Alternatively, a configuration can also be employed in which no recessed portion 14 is provided, and no adhering material is applied to a portion at which the recessed portion 14 may have been provided. With this configuration, a finger can be inserted into a portion to which no adhering material is applied.

Although the base 1 is fixed to the skin using the adhering material 12 in the above-mentioned embodiment, the adhering material 12 need not be applied to the entire lower surface of the base 1 and may be applied to a portion thereof. There is no limitation to the configuration in which an adhering material is used, and a configuration can also be employed in which the base 1 is fixed to the skin using a piece of tape or the like, for example.

There is no particular limitation on the size, shape, and thickness of the base 1 as long as the base 1 can be arranged on the skin and is provided with the through hole 11. Moreover, a configuration can also be employed in which the base 1 and the drug administration portion 2 are separately formed, and the drug administration portion 2 is fixed onto the base 1 using an adhesive or through thermal welding.

### C-6

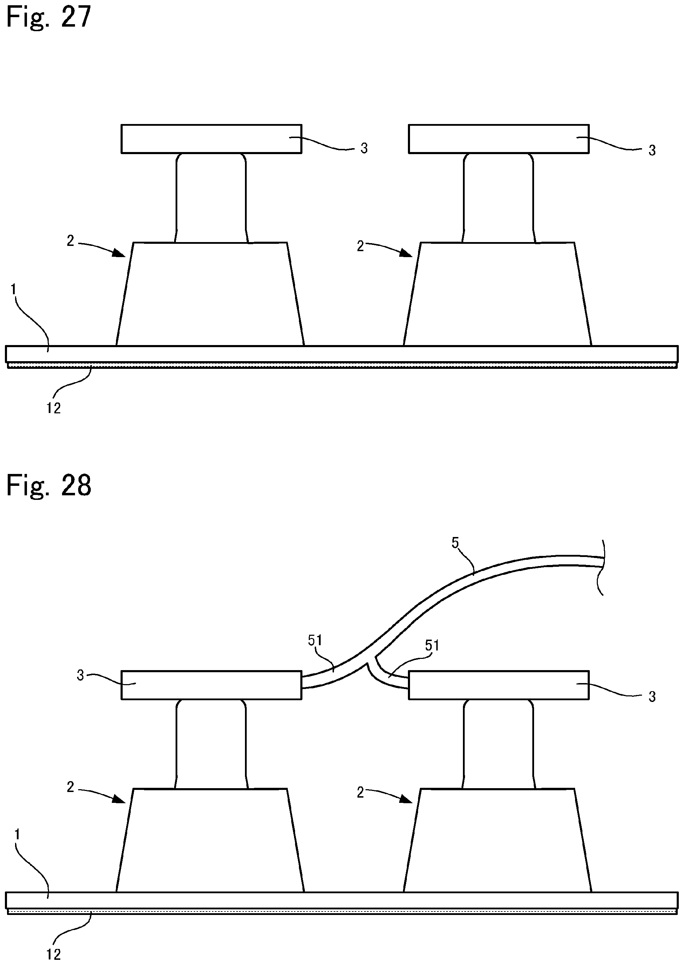

Although the base 1 is provided with a single drug administration portion 2 in the above-mentioned embodiment, a plurality of drug administration portions 2 can also be provided as shown in FIG. 27. With this configuration, a large amount of a drug can be subcutaneously administered in a short period of time.

At this time, a configuration can be employed in which the drug lines 5 are individually attached to the drug administration portions 2, or a configuration can be employed in which a plurality of branch lines 51 branched off from a single drug line 5 are individually coupled to the drug administration portions 2 as shown in FIG. 28. Moreover, a configuration can also be employed in which portions of the drug line 5 and the branch lines 51 are fixed to the base 1 and combined into a single body. With this configuration, it is possible to prevent entanglement of the lines 5 and 51, and attachment of the lines 5 and 51 to the adhering material 12.

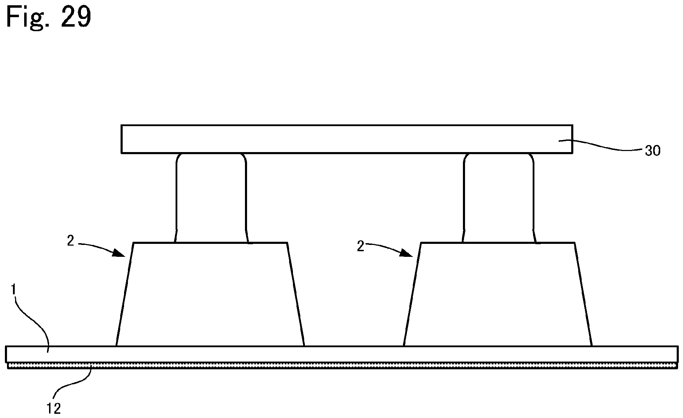

As shown in FIG. 29, a configuration can also be employed in which a single operation portion 30 is attached to a plurality of drug administration portions 2. With this configuration, all the movable portions 22 can be moved simultaneously from the first positions to the second positions by pressing the operation portion 30, and all the movable portions 22 can be moved simultaneously from the second positions to the first positions by pulling the operation portion 30. When a plurality of drug administration portions 2 are used in this manner, a plurality of needle members 4 can be provided.
Furthermore, a drug administration system can be configured by combining the drug administration device of the present invention with at least one drug container (infusion bag) containing a drug, at least one drug line that connects the drug container and the drug administration device, and the like. The drug administration system can be configured such that one or more drug administration devices that each include one or more drug administration portions 2 are used to simultaneously insert 1 to 20 needle members 4 into the skin and administer a drug for a single drug administration. The drug administration system can also be configured such that, for a single administration, 1 to 10 needle members are preferably used, and 1 to 5 needle members are more preferably used. In this case, one drug administration portion 2 can also be provided with a plurality of needle members 4 in the movable portion 22. Moreover, there is no particular limitation on the number of drug lines. As many drug lines as the drug administration portions 2 may be provided, or a branched drug line (see FIG. 28, for example) that can be connected to a plurality of drug administration portions 2 may be used. In addition, there is no particular limitation on the number of infusion bags, and a configuration may also be employed in which a drug is supplied from a plurality of infusion bags to a plurality of drug administration portions 2. When a plurality of drug administration devices are used, application sites (puncture sites) are selected from the abdominal region, the chest region, the femoral region, the dorsal region, the upper arm, and the like as appropriate, and administration can be performed in a state in which the drug administration devices are spaced apart from one another.

### C-7

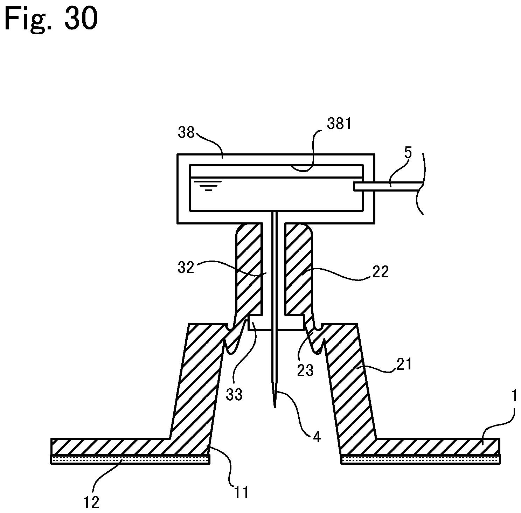

Although a drug is directly supplied from the drug line 5 to the needle member 4 in the above-mentioned embodiment, a drug storage portion 38 can also be provided between the needle member 4 and the drug line 5 as shown in FIG. 30, for example. This storage portion 38 is provided with a closed space 381 for storing a drug supplied from the drug line 5, and the needle member 4 is coupled to this closed space 381. Therefore, even if the supply of a drug from the drug line 5 is stopped for some reason, for example, the drug stored in the storage portion 38 is supplied to the needle member 4, thus making it possible to stably perform the subcutaneous administration of the drug. With such a subcutaneous administration device including the storage portion 38, it is sufficient that only a single line is provided even when a plurality of needle members are used, and thus problems such as entanglement of lines do not arise. Note that only the drug stored in the storage portion 38 can be administered without providing the drug line 5. Note that only the drug stored in the storage portion 38 can be administered without providing the storage portion 38 with the drug line 5.

### C-8

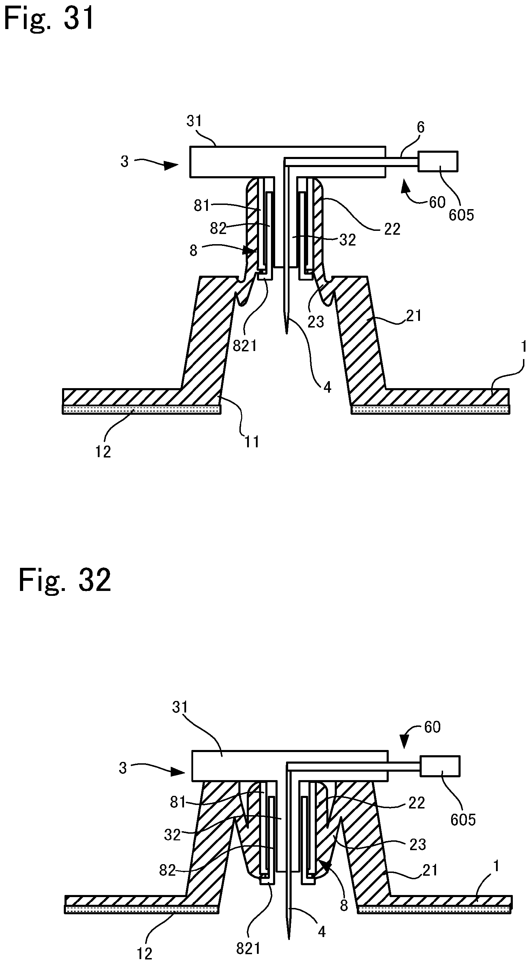

The above-mentioned embodiment can be provided with a mechanism for covering the needle in order to prevent reuse of the needle member 4 that has already been used, and mispuncture. For example, as shown in FIG. 31, a tubular cover member 8 is attached to surround the fixation portion 32 of the operation portion 3, and the operation portion 3, the needle member 4, and the cover member 8 are attached to the movable portion 22 in this state. The cover member 8 includes an outer tube 81 and an inner tube 82 that are substantially concentric and have substantially the same length in the axial direction, and the inner tube 82 is housed in the outer tube 81 in the initial state. Moreover, the outer circumferential surface of the outer tube 81 is fixed to the inside of the movable portion 22. However, when strongly pulled up, the outer tube 81 is detached from the movable portion 22. The inner tube 82 can slide along the outer tube 81 in the axial direction, and the cover member 8 includes a locking mechanism (not shown) for locking the lower end portion of the outer tube 81 to the upper end portion of the inner tube 82.

The upper end portion of the outer tube 81 is fixed to the lower surface of the basal portion 31 of the operation portion 3, and the lower end portion of the inner tube 82 slightly protrudes from the outer tube 81 and is fixed to the lower end of the movable portion 22. That is, the inner tube 82 is provided with a flange portion 821 protruding outward in the radial direction at the lower end portion, and this flange portion 821 is engaged with the lower end of the movable portion 22.

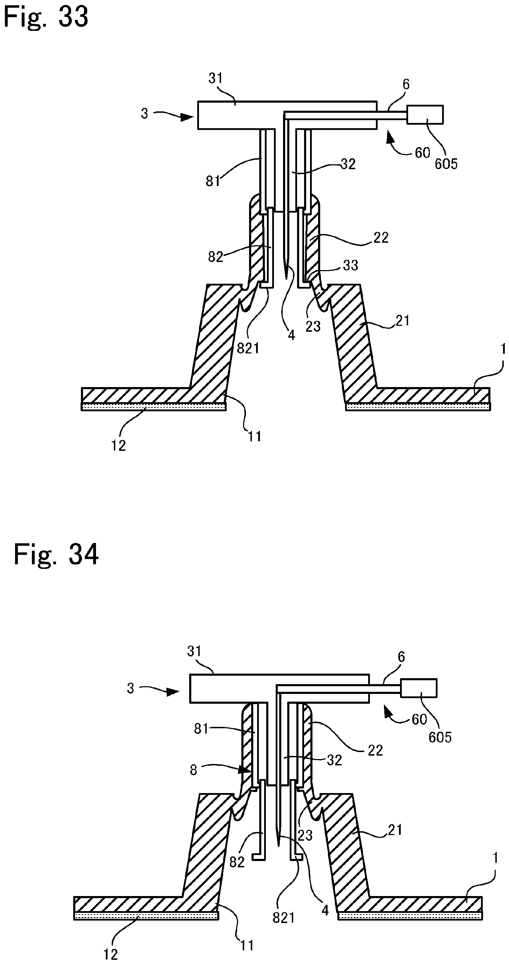

With such a configuration, when the operation portion 3 is pressed downward from the first position, the movable portion 22 is moved downward together with the cover member 8 and the needle member 4 as shown in FIG. 32, and the needle member 4 is inserted into the skin through the through hole 11 of the base 1. When the operation portion 3 is held and pulled up in this state, the movable portion 22 is moved to the first position together with the cover member 8 and the needle member 4 as shown in FIG. 31. When the operation portion 3 is further pulled up strongly in this state, the outer tube 81 is dislodged from the movable portion 22 and pulled up while sliding along the inner tube 82, as shown in FIG. 33. At this time, since the flange portion 821 of the inner tube 82 is engaged with the lower end of the movable portion 22, the inner tube 82 stays inside the movable portion 22. When the lower end of the outer tube 81 is locked to the upper end of the inner tube 82, the outer tube 81 and the inner tube 82 are coupled to each other in the axial direction, and the cover member 8 is extended in the axial direction. As a result, the needle member 4 is accommodated in the extended cover member 8 and thus ceases to be exposed to the outside. Since the outer tube 81 and the inner tube 82 are locked to each other, the extended cover member 8 does not return to the initial state even when the operation portion 3 is pushed down. Therefore, the needle member 4 remains accommodated in the extended cover member 8, thus preventing reuse thereof as well as preventing spread of infectious diseases by preventing mispuncture by the used needle member.

In the example shown in FIG. 31, when the movable portion 22 is located at the first position, the inner tube 82 is housed in the outer tube 81. However, a configuration can also be employed in which, when the movable portion 22 is located at the first position, the inner tube 82 protrudes downward from the movable portion 22 and covers the needle member 4 as shown in FIG. With this configuration, it is possible to reliably prevent patients and other users from touching the needle member 4 before use. When the operation portion 3 is pressed, the flange portion 821 of the inner tube 82 is pressed against the skin, and the inner tube 82 slides and is housed in the outer tube 81 as shown in FIG. 32. Thereafter, when the operation portion 3 is pulled up, the movable portion 22, the outer tube 81, and the inner tube 82 are pulled up in an integrated manner as shown in FIG. 31. If a configuration is employed in which the lower end portion of the outer tube 81 and the upper end portion of the inner tube 82 are locked to each other when the operation portion 3 is further pulled up, the needle member 4 will be accommodated in the inner tube 82 as shown in FIG. 33.

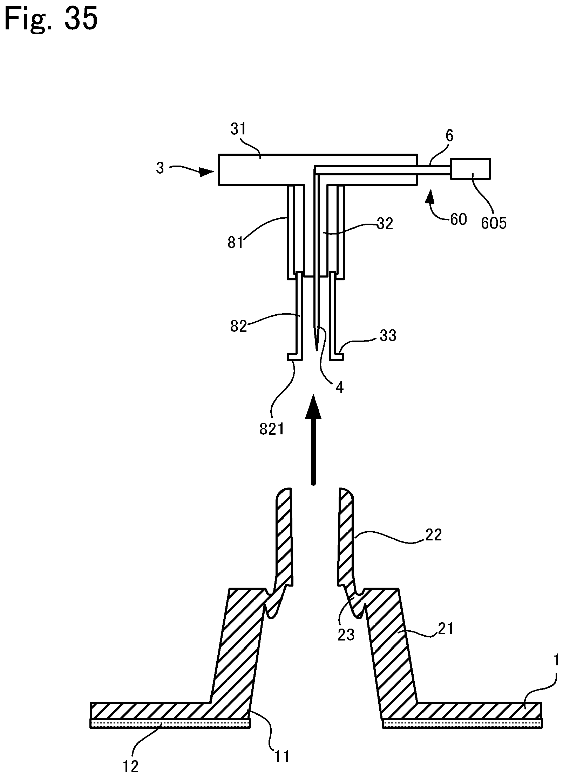

It should be noted that, when the operation portion 3 is further pulled up with a strong force, the flange portion 821 of the inner tube 82 is dislodged from the lower end of the movable portion 22, and thus the cover member 8, the needle member 4, and the operation portion 3 can be detached from the movable portion 22 as shown in FIG. 35, thus enabling separate collection. Accordingly, it is possible to prevent reuse of the drug administration device and the needle member 4 and mispuncture. At this time, the state in which the needle member 4 is accommodated in the cover member 8 can be maintained, and thus infection and the like are prevented.

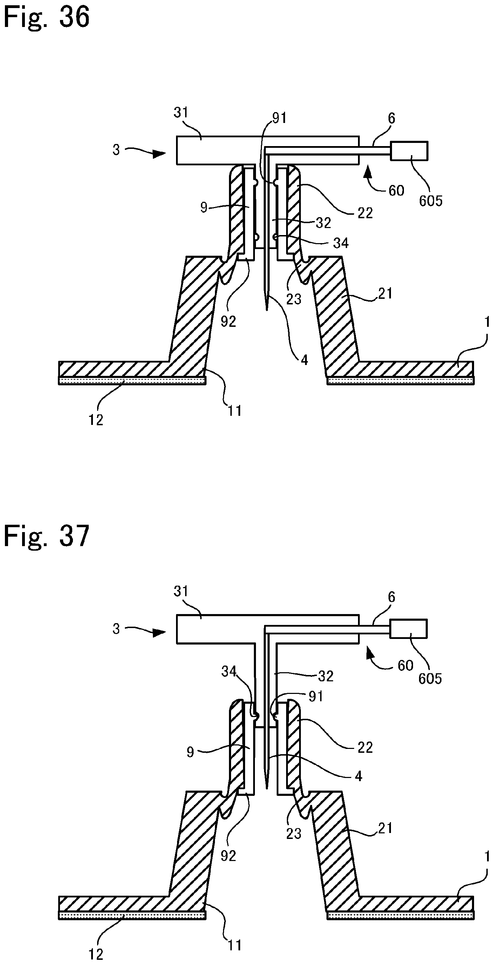

Although the cover member 8 of the aspects shown in FIGS. 31 to 35 is constituted by the inner tube 82 and the outer tube 81, a configuration can also be employed in which the cover member includes only an inner tube. For example, in the example shown in FIG. 36, a cover member 9 with a cylindrical tubular shape is fixed in the internal space of the movable portion 22. This cover member 9 is provided with a flange portion 92 at the lower end portion, and this flange portion 92 is engaged with the lower end of the movable portion 22. The cover member 9 is provided with an annular protrusion 91 on the upper portion of the inner wall surface thereof. The fixation portion 32 of the operation portion 3 is slidably inserted into the cover member 9. The fixation portion 32 is provided with an annular groove 34 on the lower portion of the outer circumferential surface thereof, and the protrusion 91 of the cover member 9 can be engaged with this groove 34.

In the configuration as described above, when the operation portion 3 is pulled up in the state shown in FIG. 36, the fixation portion 32 slides upward as shown in FIG. 37. Then, when the protrusion 91 of the cover member 9 is engaged with the groove 34 of the fixation portion 32, the operation portion 3 cannot be further pulled up. That is, a dislodgement prevention mechanism is constituted by the groove 34 and the protrusion 91 such that the fixation portion 32 is not dislodged from the cover member 9. In this state, the needle member 4 is accommodated in the cover member 9. When the operation portion 3 is strongly pulled up in this state, the flange portion 92 of the cover member 9 is dislodged from the movable portion 22, and the operation portion 3, the cover member 9, and the needle member 4 are detached from the movable portion 22 in the state in which the needle member 4 is accommodated in the cover member 9 as in the case shown in FIG. 35. Accordingly, the cover member 9, the needle member 4, and the operation portion 3 can be separately collected, and it is possible to prevent reuse of the drug administration device and the needle member 4 and mispuncture. It should be noted that there is no particular limitation on the means for engaging the pulled-up fixation portion 32 with the cover member 9, and various dislodgement prevention mechanisms and the like other than the above-described dislodgement prevention mechanism can be used.

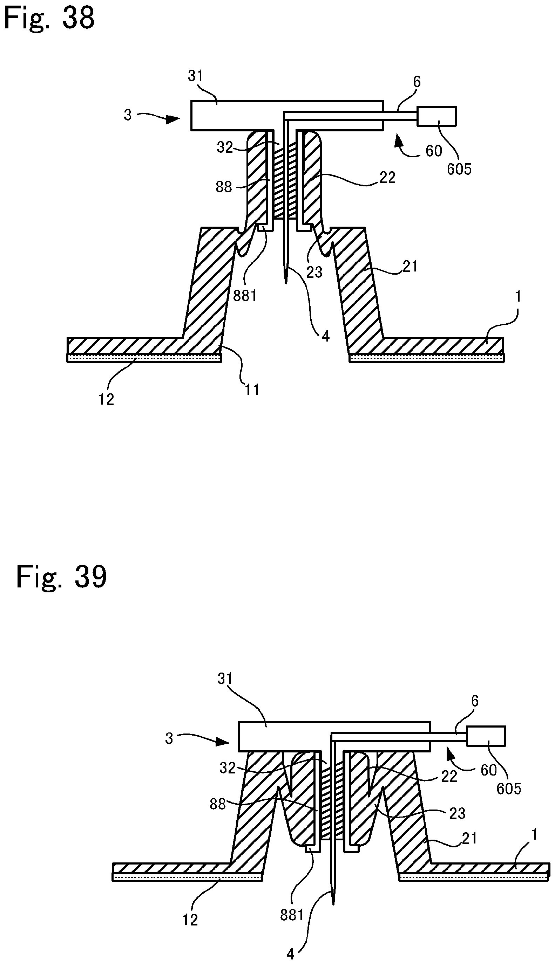

The following configuration can also be employed in order to prevent reuse of the drug administration device and the needle member 4. For example, as shown in FIG. 38, a male screw thread is formed on the outer circumferential surface of the fixation portion 32 to which the needle member 4 is attached, and the fixation portion 32 is accommodated in a cover member 88 with a cylindrical tubular shape on which a female screw thread is formed. The length of the cover member 88 in the axial direction is set to be about half of the length of the needle member 4, and the needle member 4 is made to protrude from the leading end of the cover member 88 by rotating the fixation portion 32 together with the needle member 4 around the axis. Then, the cover member 88 is fixed to the movable portion 22. However, when strongly pulled up, the cover member 88 is detached from the movable portion 22. Moreover, since the cover member 88 is provided with a flange portion 881 at the lower end portion thereof, the flange portion 881 of the cover member 88 and the lower end of the movable portion 22 can be engaged with each other.

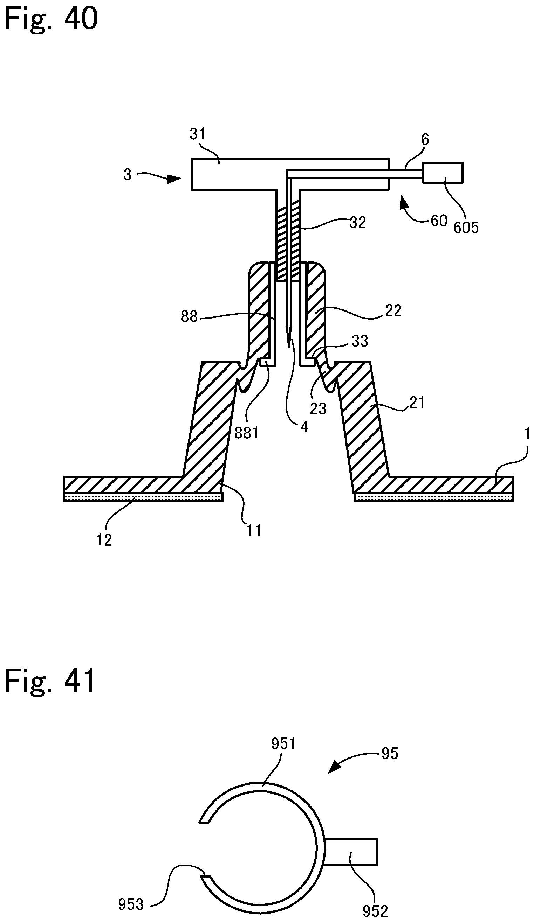

With such a drug administration device, once subcutaneous administration of a drug as shown in FIG. 39 is complete, the movable portion 22 is moved from the second position to the first position, and then the fixation portion 32 is rotated from the state shown in FIG. 38 together with the needle member 4 around the axis. Thus, as shown in FIG. 40, the needle member 4 is moved up inside the cover member 88 together with the operation portion 3 while being rotated around the axis. Then, when the leading end of the needle member 4 is completely accommodated in the cover member 88, the rotation is stopped. As a result, the state in which the needle member 4 is accommodated in the cover member 88 is maintained, and thus the needle member 4 is not exposed even when the operation portion 3 is pushed down in this state. Accordingly, reuse of the needle member 4 is prevented. It should be noted that a configuration can be also employed in which the rotation is locked when the needle member 4 is rotated until the leading end of the needle member 4 is completely accommodated in the cover member 88. When the operation portion 3 is strongly pulled up from the state shown in FIG. 40, the cover member 88 is detached from the movable portion 22, and thus the cover member 88, the needle member 4, and the operation portion 3 can be separately collected. As a result, it is possible to prevent reuse of the drug administration device and the needle member 4 and mispuncture. It should be noted that luer locks can also be used instead of screws.

### C-9

The above-mentioned embodiment can also be provided with a stopper for holding the movable portion 22 at the first position. As shown in FIG. 41, a stopper 95 includes a stopper main body 951 with a cylindrical tubular shape and a handle portion 952 attached to the outer circumferential surface of the main body 951, and is made of a resin material or the like. The stopper main body 951 is provided with a cutout portion 953 extending in the axial direction at a portion in the circumferential direction, and is formed in a C shape as a whole in a plan view. The inner diameter of the stopper main body 951 is slightly larger than the outer diameter of the movable portion 22, and is smaller than the outer diameters of the basal portion 31 of the operation portion 3 and the main body portion 21. The width of the cutout portion 953 in the circumferential direction is smaller than the outer diameter of the movable portion 22.

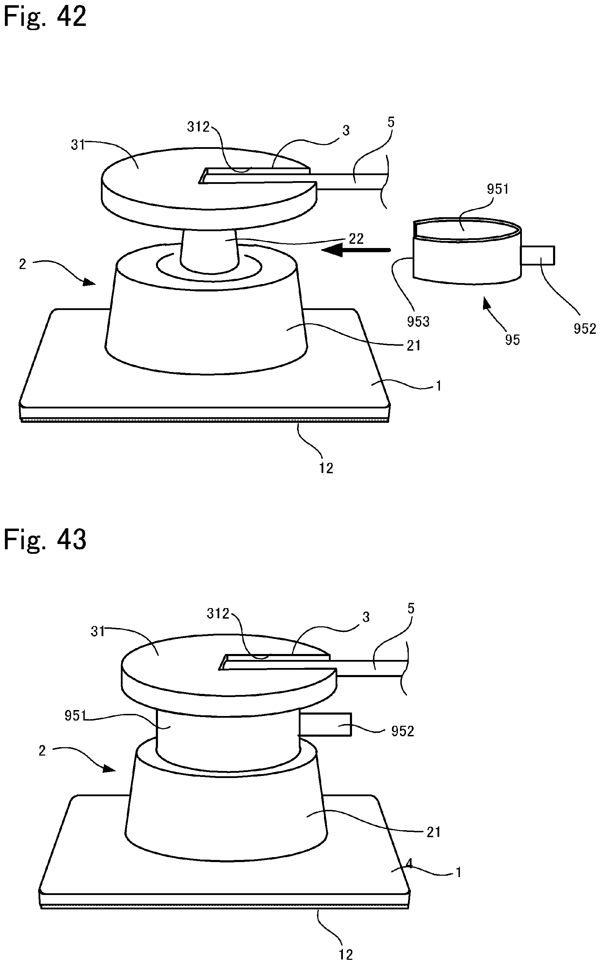

As shown in FIG. 42, the stopper 95 is inserted between the operation portion 3 and the main body portion 21 when the movable portion 22 of the drug administration device is located at the first position. Then, the cutout portion 953 of the stopper main body 951 is widened, and the stopper 95 is arranged to cover the outer circumferential surface of the movable portion 22. Thus, as shown in FIG. 43, the stopper main body 951 is arranged between the basal portion 31 of the operation portion 3 and the main body portion 21, and the operation portion 3 is restricted from moving to the main body portion 21 side. That is, the movement of the movable portion 22 from the first position to the second position is restricted. Therefore, it is possible to prevent the movable portion 22 from being accidentally moved to the second position due to an impact or load caused by something. In order to move the movable portion 22 to the second position, the handle portion 952 need only be pulled. Thus, the cutout portion 953 is widened, and the stopper main body 951 can be removed from the movable portion 22. It should be noted that there is no particular limitation on the configuration of the stopper 95, and it is sufficient that the stopper 95 is configured to be capable of being detachably arranged between the basal portion 31 of the operation portion 3 and the main body portion 21 when the movable portion 22 is located at the first position. Therefore, the operation portion 3 need not have the configuration as shown in FIG. 42. The operation portion 3 may also be configured to have a flange shape and protrude from the outer circumferential surface of the movable portion 22, and the stopper 95 can also be arranged between such an operation portion and the main body portion 21.

### C-10

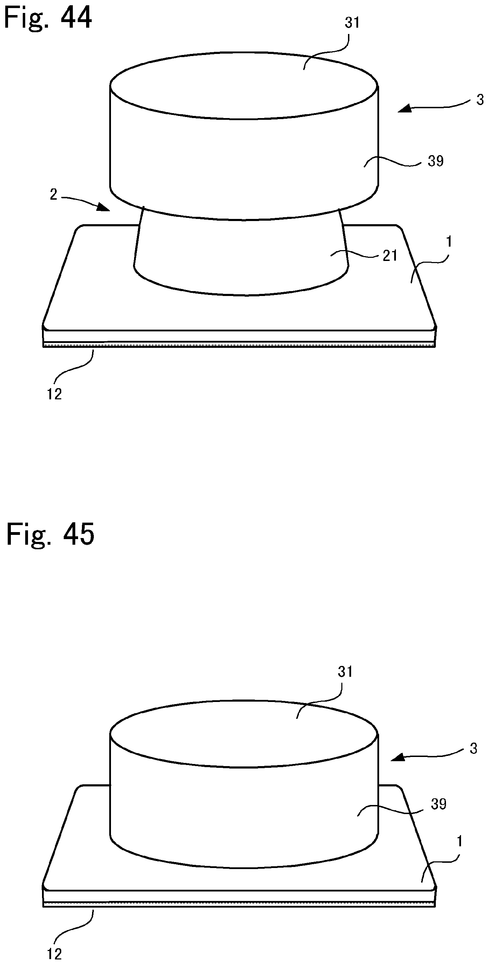

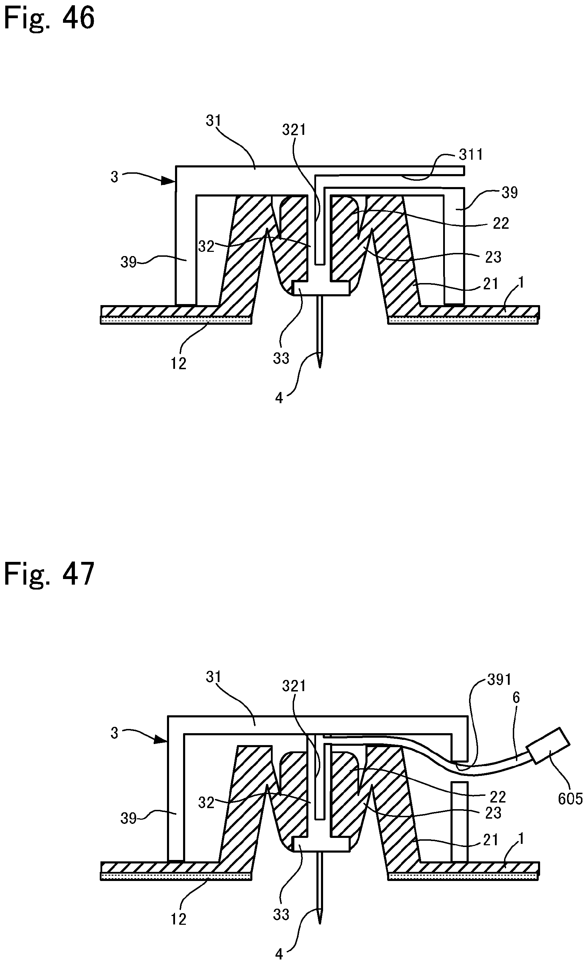

The above-mentioned embodiment can also be configured such that the state in which the movable portion 22 is located at the second position is maintained. For example, as shown in FIG. 44, the operation portion 3 can be provided with an extension portion 39 with a cylindrical tubular shape that extends downward from the circumferential edge of the basal portion 31. As shown in FIGS. 45 and 46, the extension portion 39 is configured such that its lower end portion comes into contact with the base 1 when the movable portion 22 is located at the second position. Thus, the operation portion 3 is stably held on the base 1. Accordingly, it is possible to prevent the operation portion 3 and the movable portion 22 from being shifted or lifted up when an impact or load is applied thereto in the lateral direction, for example. Therefore, it is possible to prevent the needle member 4 inserted into the skin from accidentally coming out from the skin, or to prevent the needle member 4 from moving in the state in which the needle member 4 is inserted into the skin.

It should be noted that there is no particular limitation on the configuration of the extension portion 39, and it is sufficient that the extension portion 39 is configured to be arranged between the basal portion 31 of the operation portion 3 and the base 1 when the movable portion 22 is located at the second position. The extension portion 39 need not have a cylindrical tubular shape. That is, it is sufficient that the extension portion 39 is configured such that its lower end portion is located near the lower end portion of the main body portion 21 when the movable portion 22 is located at the second position. In other words, a configuration can be employed in which at least a portion of the main body portion 21, preferably most of the main body portion 21, is covered by the extension portion 39 located at the second position. For example, the entire operation portion 3 including the basal portion 31 and the extension portion 39 may also be formed in a mushroom shape, a dome shape, or the like. In addition, in the case where the base 1 is not provided as described later, the lower end of the extension portion 39 located at the second position is located near the lower end portion of the main body portion 21 and is located near the skin or is in contact with the skin, thus making it possible to prevent the operation portion 3 from being shifted as mentioned above.

The drug line 5 for supplying a drug to the needle member 4 is coupled to the basal portion 31 of the operation portion 3. In addition, a configuration can also be employed in which the extension portion 39 is provided with a hole 391 or a cutout as shown in FIG. 47, and the tube 6 is fixed thereto so as to be coupled to the second passage 321 of the fixation portion 32, for example. Thus, it is possible to connect the connector 605 provided at one end of the tube 6 to the drug line 5 and to supply a drug to the needle member 4. It should be noted that the drug line 5 can also be directly connected to the fixation portion 32.

### C-11

In the above-mentioned embodiment, the movable portion is held at the first position due to the material, shapes, and dimensional designs of the movable portion 22, coupling portion 23, and main body portion 21. However, the present invention is not limited thereto, and a configuration may also be employed in which the movable portion 22 cannot be held at the first position when an external force is not applied thereto. In this case, the movable portion 22 can be held at the first position using the above-described stopper 95. That is, with such a drug administration device, when the stopper 95 is removed in the state shown in FIG. 43, for example, the movable portion 22 moves from the first position to the second position without applying an external force thereto, and the needle is inserted into the skin as shown in FIGS. 5A and 5B, and then administration is started. After the administration is complete, the entire drug administration device is removed from the skin, or the drug administration device can be removed from the skin after the fixation portion 32 is removed from the movable portion 22.

The above-mentioned embodiment is designed such that the force required to move the movable portion 22 from the second position to the first position (referred to as "F2" hereinafter) is larger than the force required to move the movable portion 22 from the first position to the second position (referred to as "F1" hereinafter). It should be noted that F1 is the force required to push down the movable portion 22, and F2 is the force required to pull up the movable portion 22. However, the present invention is not limited to this configuration, and the material, shape, and dimensions thereof can also be designed such that the relationship F1=F2 or F1>F2 is satisfied. That is, it is sufficient that the present invention is configured such that the movable portion 22 can be held at the first position and the second position using various methods such as changing materials as appropriate, adjusting the shapes of the members (e.g., the main body portion 21, the movable portion 22, and the coupling portion 23), and using another member such as a stopper.

### C-12

In the present invention, there is no particular limitation on the shape of the main body portion 21. Although the main body portion 21 of the above-mentioned embodiment is formed in a tubular shape whose diameter increases downward, the main body portion 21 can also be formed in a tubular shape having a constant inner diameter, a tubular shape whose diameter decreases downward, a polygonal tubular shape, or another shape. In all cases, the axial direction means a direction in which the axis of the tubular shape extends. Moreover, when the main body portion 21 has a cylindrical tubular shape, the coupling portion 23 has an annular shape, whereas when the main body portion 21 has a polygonal tubular shape, the coupling portion 23 is correspondingly formed in a polygonal shape in a plan view.

### C-13

In the present invention, there is also no particular limitation on the shape of the basal portion 31 of the operation portion 3. The basal portion 31 may be formed in a shape other than a disk shape, namely a rectangular shape, a polygonal shape, or another shape. The external size of the basal portion 31 of the operation portion 3 may be smaller than or equal to that of the end portion (first end portion) on the upper side of the main body portion 21. Thus, at the second position, the basal portion 31 does not protrude from the main body portion 21 in the radial direction, and therefore, when this drug administration device is attached to the skin, it is possible to reduce the risk of the drug administration device being detached from the skin due to the basal portion 31 rubbing against or being caught on clothes.

### C-14

It is sufficient that the above-mentioned drug administration device includes at least the main body portion 21, the coupling portion 23, the movable portion 22, and the needle member 4, and is configured such that the drug line 5 can be directly or indirectly connected to the needle member 4. That is, the base 1 need not be provided, and a configuration can also be employed in which the end portion (second end portion) of the main body portion 21 in the axial direction is directly attached to the skin. Moreover, as shown in FIG. 26, a configuration can also be employed in which the main body portion 21 is provided with a recessed portion at the lower end portion to facilitate removal from the skin. In this case, an adhering material can also be applied to the end portion of the main body portion 21 excluding the recessed portion, or an adhering sheet or seal can also be arranged thereon. Therefore, the presence or absence of the operation portion 3 as well as the shapes, material, and the like of the members can be changed as appropriate. For example, there is no particular limitation on the material of the main body portion 21, the movable portion 22, and the coupling portion 23 as long as the movable portion 22 can be moved between the first position and the second position. Therefore, examples of the material forming these members include various materials, namely the above-described soft resin materials such as elastomer as well as resin materials such as hard resin (e.g., polypropylene, polyethylene, and polyethylene terephthalate), and metals. The main body portion 21, the movable portion 22, and the coupling portion 23 are not necessarily formed as a single body and can be formed by attaching a plurality of materials to each other.

Although, in the above-mentioned embodiments, the movable portion 22 is accommodated in the main body portion 21 when located at second position, it does not necessarily have to be accommodated therein. That is, the movable portion 22 may also be provided outside the main body portion 21 as long as the needle member 4 is inserted into the skin when the movable portion 22 is located at the second position. In this case, the shape of the coupling portion 23 is adjusted, and the coupling portion 23 is formed in an elongated shape such that the movable portion 22 is arranged outside the main body portion 21 when located at the second position. However, the size can be reduced if the movable portion 22 is accommodated in the main body portion 21 when located at the second position, and therefore, it is preferable that at least a portion of the movable portion 22 is accommodated in the main body portion 21. There is no particular limitation on the shape of the leading end of the needle member, and the leading end of the needle member can be formed in a known shape.

### C-15

The above-mentioned drug administration device can be provided with a guide member (not shown) for moving the movable portion 22 (or the needle member 4) substantially orthogonally relative to the skin around the movable portion 22 in order to move the movable portion 22 from the first position to the second position orthogonally relative to the skin, in other words, to orthogonally insert the needle member 4 into the skin. The guide member can be provided in the internal space of the main body portion 21, for example.

### C-16

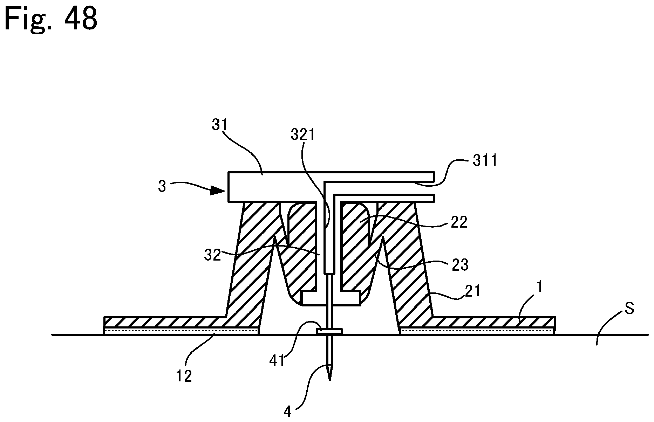

The above-mentioned drug administration device can be provided with a flange portion 41 on the outer circumferential surface of the needle member 4 as shown in FIG. 48, for example, in order to prevent the needle member 4 from staying in the skin due to the needle being broken at the time of puncture. This flange portion 41 can be provided in a region of the needle member that is located between the fixation portion 32 and the skin S and exposed to the outside when the movable portion 22 is located at the second position. Thus, even if the needle is broken at the time of puncture and the leading end of the needle member 4 stays in the skin S, the needle member 4 can be easily removed from the skin S by holding the flange portion 41 because the flange portion 41 is located outside the skin S. By determining the position of the flange portion 41 such that the flange portion 41 is in contact with the skin at the time of puncture, the length between the flange portion 41 and the leading end of the needle member 4 corresponds to the insertion length, thus making it possible to strictly control the insertion length. It should be noted that the flange portion 41 need not be provided over the entire outer circumferential surface of the needle member 4, and may also be a member that protrudes outward from a portion of the outer circumferential surface of the needle member 4 in the radial direction. Furthermore, when the flange portion 41 protrudes outward from a portion of the outer circumferential surface of the needle member 4 in the radial direction, the flange portion 41 is provided at a position on the needle member 4 near the skin S as shown in FIG. 48, but there is no limitation to this position. For example, the flange portion 41 can also be provided at a position near the movable portion 22 or the fixation portion 32 of the operation portion 3. That is, the flange portion 41 need only be provided between the skin S and the fixation portion 32.

### C-17

The movement of the movable portion can be controlled by forming the coupling member using an elastic member such as a plate spring. Some aspects will be described below.

### C-17-1

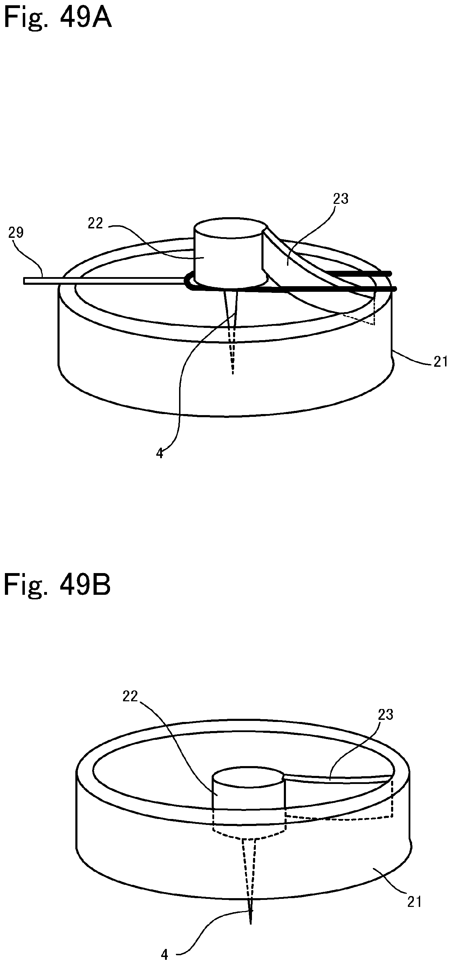

An example of the drug administration device shown in FIGS. 49A and 49B includes a main body portion 21 with a cylindrical tubular shape, and a movable portion 22 to which a needle member 4 that can be moved up and down inside the main body portion 21 is attached. These portions can be formed of a resin material such as hard resin as a single body or can be formed by combining separate materials. The upper end portion of the inner wall surface of the main body portion 21 and the movable portion 22 are coupled to each other via a coupling portion 23 that is formed of an elastic member with a plate spring shape. The following is a more detailed description. As shown in FIG. 49B, when the movable portion 22 enters the inside of the main body portion 21 and is located at a second position where the needle member 4 protrudes from the lower opening of the main body portion 21, the coupling portion 23 extends horizontally with no load being applied thereto. On the other hand, as shown in FIG. 49A, when the movable portion 22 is moved up such that the needle member 4 is spaced apart from the skin, and is located at a first position, the coupling portion 23 is bent upward, and a load is applied so as to bias the movable portion 22 downward. Then, in order to hold the movable portion 22 at the first position shown in FIG. 49A, a stopper 29 is detachably attached to the lower end portion of the movable portion 22. The stopper 29 is a member configured to span portions at the upper end of the main body portion 21 that are opposite to each other with the movable portion 22 being located therebetween while passing directly underneath the lower end of the movable portion 22, and is used to restrict downward movement of the movable portion 22. It should be noted that there is no particular limitation on the configuration of the stopper, and it is sufficient that the stopper can hold the movable portion 22 at the first position.

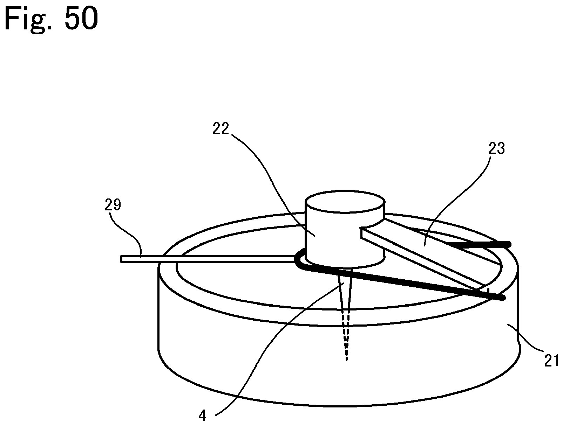

When the stopper 29 is removed from the movable portion 22 located at the first position, the movable portion 22 is biased downward by the coupling portion 23 and is moved to the second position as shown in FIG. 49B. Therefore, by attaching the lower opening of the main body portion 21 to the skin in the state in which the movable portion 22 is located at the first position, and then removing the stopper in this state, the movable portion 22 is moved down, thus making it possible to insert the needle member 4 into the skin. With the configuration shown in FIG. 49A and 49B, the movable portion 22 is moved up and down while bound to the coupling portion 23 with a plate spring shape, thus making it possible to stabilize strokes at the time of puncture. Therefore, it is possible to more precisely insert the needle member 4 to a target puncture position. Since the plate spring is arranged extending in the vertical direction, a strong biasing force is obtained, thus making it possible to firmly insert the needle member 4 into the skin. It should be noted that there is no particular limitation on the form of the elastic member. For example, when the plate spring constituting the coupling portion 23 is arranged extending in the horizontal direction as shown in FIG. 50, the biasing force generated by the spring decreases, but a load applied to the coupling portion 23 itself is also weak because the force for holding the movable portion 22 at the first position is also weak. Accordingly, problems such as breakage of the coupling portion 23 and damage to the main body portion 21 are less likely to arise.

### C-17-2

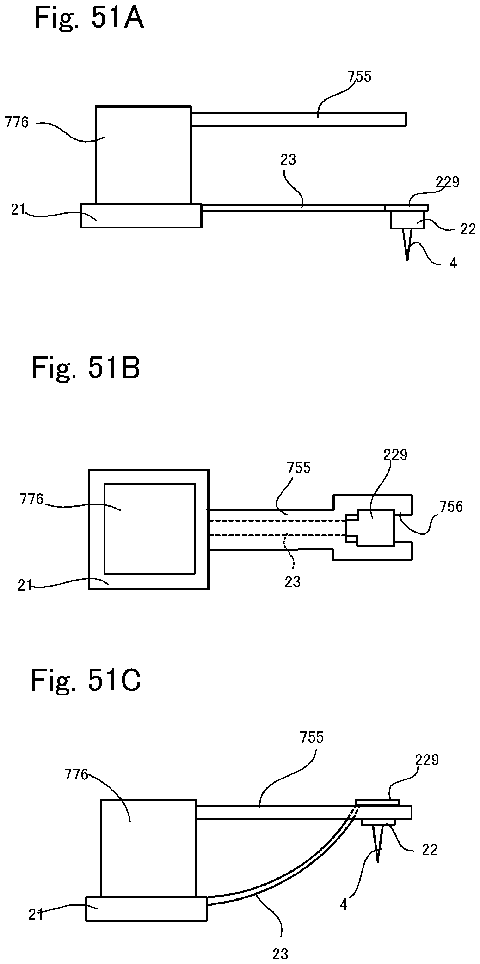

The present invention can also be configured such that the movable portion 22 is moved outside the main body portion. For example, an example shown in FIGS. 51A-51C includes a main body portion 21 with a plate shape, a coupling portion 23 constituted by an elastic member with a plate spring shape that extends from the lower end portion of the main body portion 21 in the horizontal direction, and a movable portion 22 that is attached to the leading end portion of the coupling portion 23. These portions can be formed of a resin material such as hard resin as a single body or can be formed by combining separate materials. The movable portion 22 is formed in a cylindrical tubular shape, and is provided with a flange portion 229 protruding in the horizontal direction on the upper surface. A needle member 4 protrudes downward from the lower surface of the movable portion 22. Furthermore, a base portion 776 with a rectangular parallelepiped shape is attached to the upper surface of the main body portion 21, and a holding member 755 extending in the horizontal direction is attached to the side surface of the upper end of the base portion 776. The holding member 755 is arranged above the coupling portion 23, and is provided with a cutout 756 at the leading end portion.

FIG. 51A illustrates a state in which the movable portion 22 is located at a second position. In this state, the coupling portion 23 extends horizontally, and no load is applied thereto. At this time, the needle member 4 is inserted into the skin. Then, as shown in FIGS. 51B and 51C, the movable portion 22 is moved up, and the flange portion 229 of the movable portion 22 is hooked onto the cutout 756 of the holding member 755. At this time, the coupling portion 23 is bent upward and an elastic force is applied thereto, but the movable portion 22 is hooked on the holding member 755 and held at the first position. When the base portion 776 is removed from the main body portion 21 together with the holding member 755 in this state, the movable portion 22 fixed to the holding member 755 is released. Then, as shown in FIG. 51A, the coupling portion 23 extends horizontally, and the needle member 4 is inserted into the skin. It should be noted that the needle member 4 may be inserted into the skin by merely removing the movable portion 22 from the holding member 755 while the state in which the base portion 776 is attached to the main body portion 21 is maintained.

With the configuration as described above, the main body portion 21 and the movable portion 22 are spaced apart from each other, and therefore, when there is a problem such as inflammation of a portion of the skin into which the needle member 4 is to be inserted, for example, the main body portion 21 can be attached to a portion that does not have such a problem. Accordingly, when there is a problem with the skin, the main body portion 21, namely this device, can be attached to a portion other than a portion with the problem. In addition, with the configuration as described above, the base portion 776 is removed together with the holding member 755 at the time of puncture (second position), and therefore, the overall height of the device can be reduced, thus making it possible to mitigate problems such as dislodgement of the device at the time of puncture. It should be noted that there is no particular limitation on the configurations of the main body portion, the coupling portion 23, and the holding member 755, and these configurations can be changed as appropriate. Accordingly, the coupling portion 23 and the holding member 755 can be exchanged, for example. That is, a configuration can be employed in which the coupling portion 23 is attached to the upper end of the base portion 776, and the holding member 755 is attached to the main body portion 21. With this configuration, it is possible to move the movable portion 22 down while bending the coupling portion 23 downward, and hook the movable portion 22 on the holding member 755 arranged on the lower side.

### C-18

A configuration can also be employed in which the coupling portion 23 is formed of an elastic member having a restoring force, such as a piece of rubber or a coil spring, and movement of the movable portion 22 is controlled by coupling the main body portion 21 and the movable portion 22 to each other using such an elastic member. Some examples will be described below.

### C-18-1

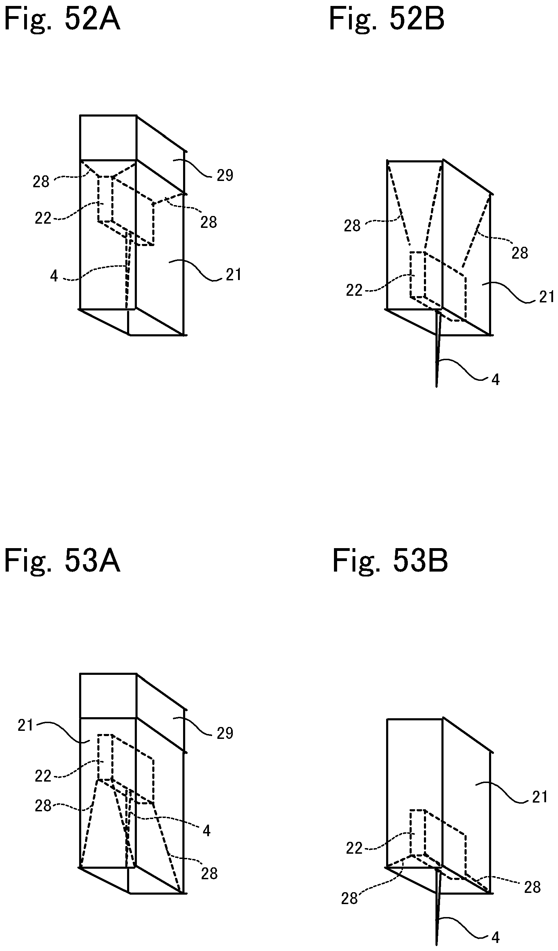

An example of the drug administration device shown in FIGS. 52A and 52B includes a hollow main body portion 21 with a rectangular parallelepiped shape whose upper and lower portions are open, and a movable portion 22 to which a needle member 4 is attached is accommodated in the main body portion 21. In the main body portion 21, an elastic member 28 such as a spring is attached coupling the upper portion of the movable portion 22 and the upper portion of the main body portion 21 to each other. The movable portion 22 is configured to be capable of being located at a first position where the needle member 4 is accommodated in the main body portion 21 as shown in FIG. 52A and at a second position where the needle member 4 protrudes from the lower opening of the main body portion 21 as shown in FIG. 52B. When the movable portion 22 is located at the first position, the elastic member 28 is compressed, and the movable portion 22 and the needle member 4 are held in the main body portion 21 by a stopper 29 that is detachably attached to the upper portion of the main body portion 21. When the stopper 29 is removed in this state, the compressed elastic member 28 extends, and thus the movable portion 22 is moved down. As a result, as mentioned above, the needle member 4 protrudes from the lower opening of the main body portion 21. Accordingly, by attaching the lower opening of the main body portion 21 to the skin in the state in which the movable portion 22 is located at the first position, and then removing the stopper 29 in this state, it is possible to insert the needle member 4 into the skin. It should be noted that the movable portion 22 and the stopper 29 can be detachably fixed to each other using a known method. Further, the main body portion 21, the movable portion 22, and the needle member 4 shown here correspond to the drug administration portion of the present invention, and the elastic member 28 in this example is also included in the drug administration portion.

### C-18-2

An example of the drug administration device shown in FIGS. 53A and 53B includes a hollow main body portion 21 with a rectangular parallelepiped shape whose upper and lower portions are open, and a movable portion 22 to which a needle member 4 is attached is accommodated in the main body portion 21. An elastic member 28 formed of a spring or rubber is attached coupling the lower portion of the movable portion 22 and the lower portion of the main body portion 21. The movable portion 22 is configured to be capable of being located at a first position where the needle member 4 is accommodated in the main body portion 21 as shown in FIG. 53A and at a second position where the needle member 4 protrudes from the lower opening of the main body portion 21 as shown in FIG. 53B.

When the movable portion 22 is located at the first position, the elastic member 28 is extended, and the movable portion 22 and the needle member 4 are held in the main body portion 21 by a stopper 29 that is detachably attached to the upper portion of the main body portion 21. When the stopper 29 is removed in this state, the extended elastic member 28 contracts, and thus the movable portion 22 is moved down. As a result, as mentioned above, the needle member 4 protrudes from the lower opening of the main body portion 21. Accordingly, by attaching the lower opening of the main body portion 21 to the skin in the state in which the movable portion 22 is located at the first position, and then removing the stopper 29 in this state, it is possible to insert the needle member 4 into the skin. It should be noted that the movable portion 22 and the stopper 29 can be detachably fixed to each other using a known method.

### C-18-3

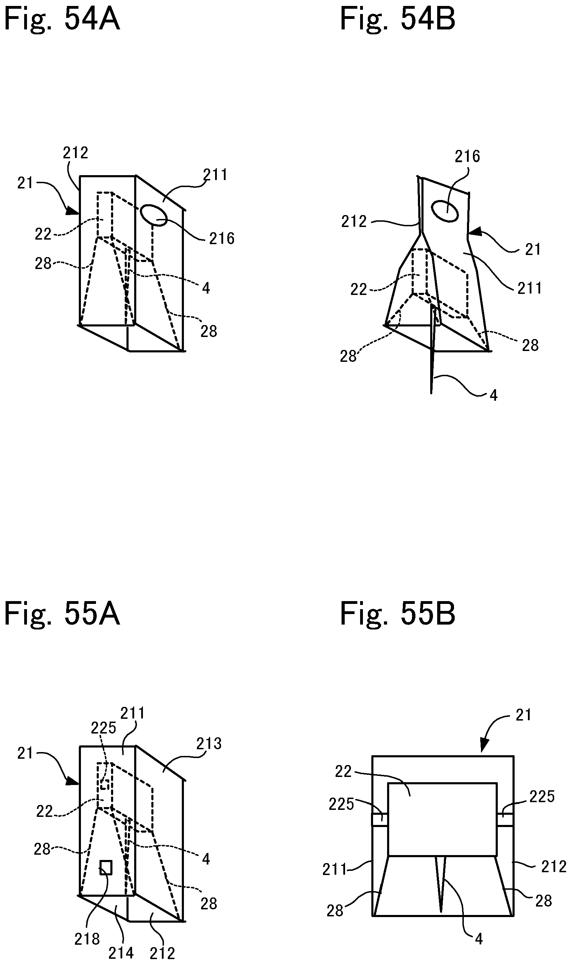

An example of the drug administration device shown in FIGS. 54A and 54B includes a hollow main body portion 21 with a rectangular parallelepiped shape whose lower portion is open, and a movable portion 22 to which a needle member 4 is attached is accommodated in the main body portion 21. An elastic member 28 such as a spring is attached coupling the lower portion of the movable portion 22 and the lower portion of the main body portion 21. Furthermore, the upper portion of the main body portion 21 can be elastically deformed, and each opposing surface 211 and 212 of the main body portion is provided with a fastener 216. The fasteners 216 can be fixed to each other by pressing the opposing surfaces 211 and 212 together. The movable portion 22 is configured to be capable of being located at a first position where the needle member 4 is accommodated in the main body portion 21 as shown in FIG. 54A and at a second position where the needle member 4 protrudes from the lower opening of the main body portion 21 as shown in FIG. 54B. Each of the above-described fasteners 216 is arranged at a position that is opposite to the vicinity of the upper end of the movable portion 22 located at the first position.

When the movable portion 22 is located at the first position, the elastic member 28 is at a substantially natural length, and no load is applied thereto. When the fasteners 216 on the opposing surfaces 211 and 212 of the main body portion 21 are pressed in this state as mentioned above, the movable portion 22 is pushed downward and moved to the second position. When the opposing surfaces 211 and 212 of the main body portion 21 come into contact with each other by fixing the fasteners 216 to each other, the internal space of the main body portion 21 is reduced above the movable portion 22 located at the second position, and thus the upward movement of the movable portion 22 is restricted. Accordingly, the movable portion 22 is held at the second position. At this time, the elastic member 28 is compressed, and the movable portion 22 is biased upward by the elastic member 28. However, since the opposing surfaces 211 and 212 of the main body portion 21 are fixed to each other by the fasteners 216 as mentioned above, upward movement is restricted. Accordingly, by attaching the lower opening of the main body portion 21 to the skin in the state in which the movable portion 22 is located at the first position, and then pressing the opposing surfaces of the main body portion 21 in this state to move the movable portion 22 to the second position, it is possible to insert the needle member 4 into the skin.

### C-18-4

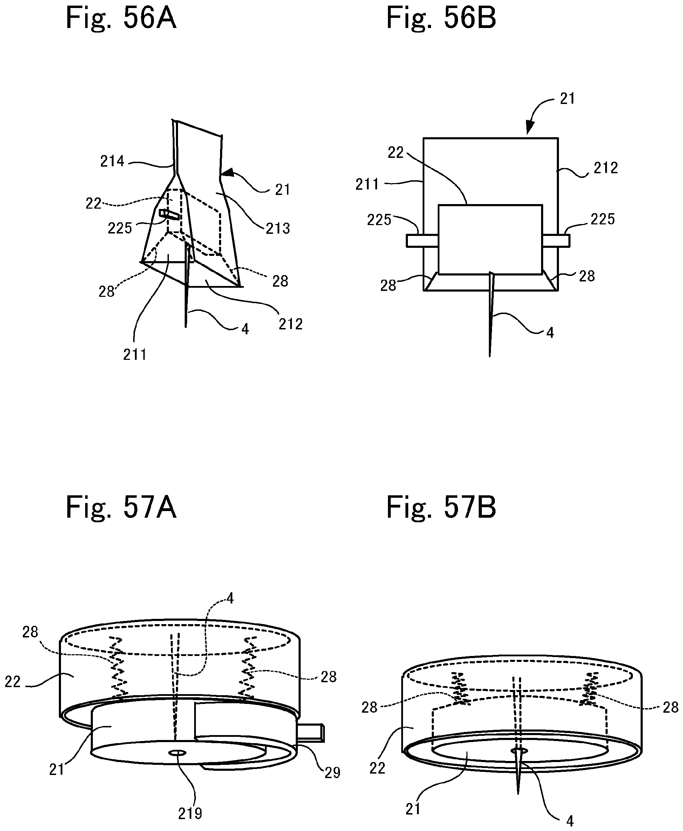

An example of the drug administration device shown in FIGS. 55A and 55B includes a hollow main body portion 21 with a rectangular parallelepiped shape whose lower portion is open, and a movable portion 22 to which a needle member 4 is attached is accommodated in the main body portion 21. An elastic member 28 such as a spring is attached coupling the lower portion of the movable portion 22 and the lower portion of the main body portion 21. Furthermore, the upper portion of the main body portion 21 can be elastically deformed. The movable portion 22 is configured to be capable of being located at a first position where the needle member 4 is accommodated in the main body portion 21 as shown in FIGS. 55A and 55B and at a second position where the needle member 4 protrudes from the lower opening of the main body portion 21 as shown in FIGS. 56A and 56B. In addition, protrusions 225 protrude from two side surfaces of the movable portion 22. The protrusions 225 protrude from the two side surfaces of the movable portion 22 due to springs (not shown). However, when the movable portion 22 is located at the first position, the protrusions 225 are respectively in contact with a first side surface 211 and a second side surface 212 of the main body portion 21 that are each opposite to the corresponding protrusion 225, and thus further protrusion is restricted. Moreover, each of the first side surface 211 and the second side surface 212 of the main body portion 21 is provided with a through hole 218, and the protrusions 225 protrude through the through holes 218 when the movable portion 22 is located at the second position. That is, when the movable portion 22 is located at the second position, the protrusions 225 are pushed outward by the springs and thus protrude through the through holes 218. Thus, the movable portion 22 is held at the second position.

When the movable portion 22 is located at the first position, the elastic member 28 is at a substantially natural length, and no load is applied thereto. When a third side surface 213 and a fourth side surface 214 of the main body portion 21 that are orthogonal to the first side surface 211 and the second side surface 212 are pressed in this state, the third side surface 213 and the fourth side surface 214 approach each other, and thus the movable portion 22 is pushed downward and moved to the second position. When the movable portion 22 is moved to the second position, the protrusions 225 protrude through the through holes 218 as mentioned above, and thus the movable portion 22 is held at the second position. At this time, the elastic member 28 is compressed, and the movable portion 22 is biased upward by the elastic member 28. However, since the protrusions 225 protrude through the through holes 218 as mentioned above, upward movement of the movable portion 22 is restricted. Accordingly, by attaching the lower opening of the main body portion 21 to the skin in the state in which the movable portion 22 is located at the first position, and then pressing the third side surface 213 and the fourth side surface 214 of the main body portion 21 in this state, it is possible to insert the needle member 4 into the skin.

### C-18-5

An example of the drug administration device shown in FIGS. 57A and 57B includes a movable portion 22 with a cylindrical tubular shape whose lower portion is open, and a needle member 4 extending downward is attached to the upper surface of the internal space of the movable portion 22. The needle member 4 protrudes from the lower opening of the movable portion 22. A cylindrical main body portion 21 can be accommodated in the movable portion 22, and elastic members 28 such as springs are attached coupling the upper portion of the main body portion 21 and the upper surface of the internal space of the movable portion 22. The main body portion 21 is provided with a through hole 219 extending in the vertical direction, and the needle member 4 is inserted through the through hole 219. The movable portion 22 is configured to be capable of being located at a first position located above the main body portion 21 as shown in FIG. 57A and being moved down to a second position where the main body portion 21 is accommodated in the internal space of the movable portion 22 as shown in FIG. 57B. When the movable portion 22 is located at the first position, the elastic members 28 are extended, and the needle member 4 is accommodated in the through hole 219 of the main body portion 21 and does not protrude from the lower portion of the main body portion 21. A stopper 29 is attached to the outer circumferential surface of the main body portion 21 in order to hold the movable portion 22 at the first position, and thereby the movable portion 22 is held so as not to move downward.

The main body portion 21 can be provided with an adhering material for fixation to the skin on the lower surface thereof. When the stopper 29 is removed in the state in which the movable portion 22 is located at the first position after the main body portion 21 is adhered to the skin with this adhering material, the elastic members 28 contract and thus the movable portion 22 is pulled down as shown in FIG. 57B. Thus, the needle member 4 protrudes downward through the through hole 219 of the main body portion 21. Accordingly, by attaching the lower surface of the main body portion 21 to the skin in the state in which the movable portion 22 is located at the first position, and then removing the stopper 29 in this state, the needle member 4 protrudes from the main body portion 21, thus making it possible to insert the needle member 4 into the skin. It should be noted that the adhering material is not necessarily used to fix the main body portion 21 to the skin, and a piece of tape as described above can also be used.

### C-18-6

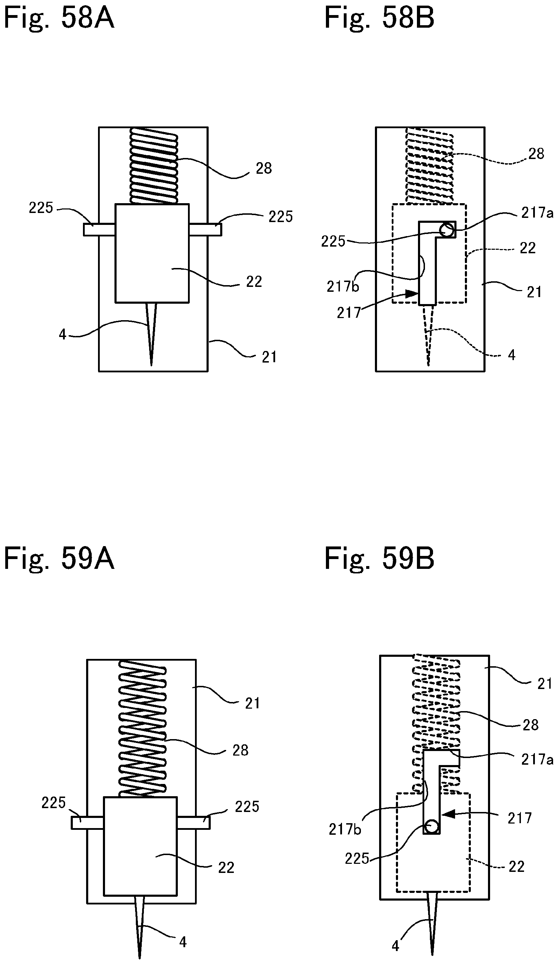

An example of the drug administration device shown in FIGS. 58A and 58B and FIGS. 59A and 59B includes a main body portion 21 with a cylindrical tubular shape whose lower portion is open, and a movable portion 22 with a cylindrical tubular shape to which a needle member 4 is attached is accommodated in the main body portion 21. An elastic member 28 such as a spring is attached coupling the upper portion of the movable portion 22 and the upper surface of the internal space of the main body portion 21. Thus, the movable portion 22 is biased downward by the elastic member 28. The movable portion 22 is provided with protrusions 225 on two opposing surfaces. The main body portion 21 is provided with slits 217 in two opposing side surfaces, and the two protrusions 225 of the movable portion 22 protrude toward the outside of the main body portion 21 through the slits 217. Each of the slits 217 is formed in an L shape including a first portion 217a extending in the horizontal direction and a second portion 217b extending downward from one end portion of the first portion 217a. The movable portion 22 is configured to be capable of being located at a first position where the needle member 4 is accommodated in the main body portion 21 as shown in FIGS. 58A and 58B and at a second position where the needle member 4 protrudes from the lower opening of the main body portion 21 as shown in FIGS. 59A and 59B.

When the movable portion 22 is located at the first position, the elastic member 28 is compressed, and the protrusions 225 are arranged in the first portions 217a of the slits 217. Accordingly, the downward movement of the protrusions 225 is restricted, and thus the movable portion 22 is held at the first position. When the protrusions 225 are moved in the horizontal direction to the upper ends of the second portions 217b of the slits 217 in this state by rotating the movable portion 22 with a cylindrical tubular shape, the protrusions 225 can move downward along the second portions 217b. Thus, the movable portion 22 is pushed down by the elastic member 28 and is moved to the second position. When the protrusions 225 are moved to the lower end portions of the second portions 217b, further downward movement is restricted, and therefore, the length of a portion of the needle member 4 protruding from the main body portion 21 is kept constant. Accordingly, by attaching the lower opening of the main body portion 21 to the skin in the state in which the movable portion 22 is located at the first position, and then moving the protrusions 225 horizontally in this state, the movable portion 22 is moved downward, thus making it possible to insert the needle member 4 into the skin.

### C-18-7

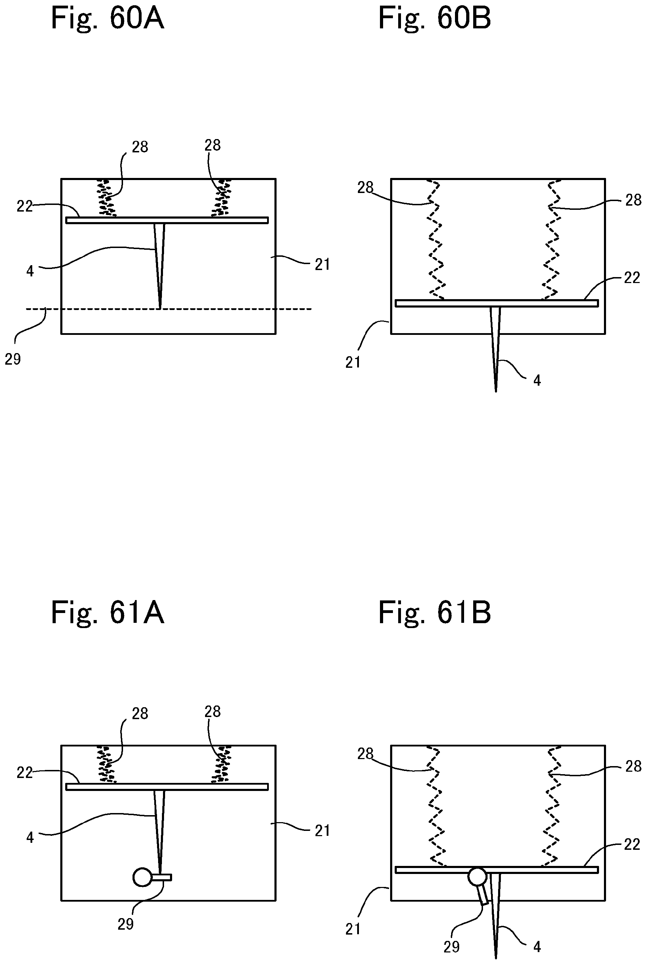

An example of the drug administration device shown in FIGS. 60A and 60B includes a hollow main body portion 21 with a rectangular parallelepiped shape whose lower portion is open, and a movable portion 22 to which a needle member 4 is attached is accommodated in the main body portion 21. Elastic members 28 such as springs are attached coupling the upper portion of the movable portion 22 and the upper portion of the internal space of the main body portion 21. Thus, the movable portion 22 is biased downward by the elastic members 28. A stopper 29 with a plate shape for holding the needle member 4 is detachably attached to the lower end portion of the main body portion 21. The movable portion 22 is configured to be capable of being located at a first position where the needle member 4 is accommodated in the main body portion 21 as shown in FIG. 60A and at a second position where the needle member 4 protrudes from the lower opening of the main body portion 21 as shown in FIG. 60B.

When the movable portion 22 is located at the first position, the elastic members 28 are compressed, the leading end of the needle member 4 is in contact with the stopper 29, and downward movement of the needle member 4 and the movable portion 22 is restricted. When the stopper 29 is removed in this state, the movable portion 22 is pushed down by the elastic members 28 and is moved to the second position. Accordingly, by attaching the lower opening of the main body portion 21 to the skin in the state in which the movable portion 22 is located at the first position, and then removing the stopper 29 in this state, the movable portion 22 is moved downward, thus making it possible to insert the needle member 4 into the skin.

### C-18-8

In the drug administration device shown in FIGS. 60A and 60B, there is no particular limitation on the configuration of the stopper, and known stoppers in various forms can be used. For example, as shown in FIGS. 61A and 61B, provided is a stopper 29 that is in contact with the leading end of the needle member 4 when the movable portion 22 is located at the first position (FIG. 61A). The stopper 29 is rotatable, and can be rotated by an operation performed outside the main body portion 21. Accordingly, by rotating the stopper 29 in the state in which the needle member 4 is located at the first position, and then releasing the contact with the needle member 4, the movable portion 22 is pushed down by the elastic members 28 and is moved to the second position (FIG. 61B).

### C-18-9

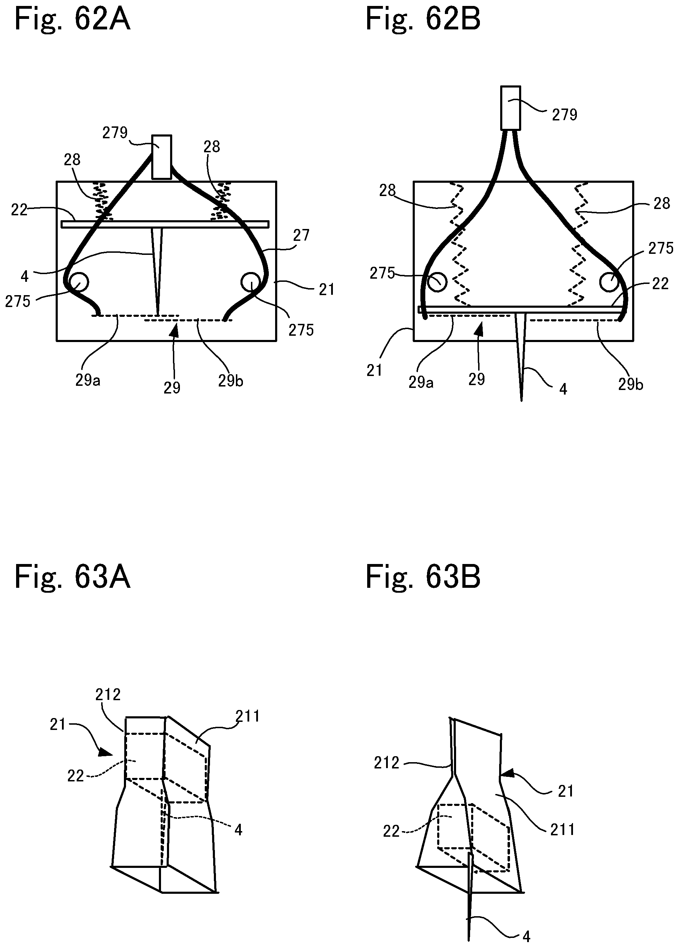

As shown in FIGS. 62A and 62B, a stopper 29 constituted by two parts is provided in the lower portion of a main body portion 21. Two parts 29a and 29b can be moved in the horizontal direction. When the two parts 29a and 29b approach and overlap each other, a needle member 4 located at the first position is in contact therewith. Thus, a movable portion 22 shown in FIG. 62A is held at the first position. Two end portions of a thread member 27 are respectively fixed to the parts 29a and 29b of the stopper 29. The thread member 27 is hooked on rollers 275 provided on the two sides of the main body portion 21, and the intermediate portion of the thread member 27 protrudes upward from the upper portion of the main body portion 21. This protrusion is provided with a fixture 279.

With this configuration, when the fixture 279 is pulled up, the thread member 27 is also pulled up. Thus, the parts 29a and 29b of the stopper 29 fixed to the two ends of the thread member 27 are moved so as to be spaced apart from each other. As a result, the contact between the stopper 29 and the needle member 4 shown in FIG. 62B is released. Thus, the movable portion 22 is moved down, and the needle member 4 protrudes from the main body portion 21.

### C-19

Movement of a movable portion can also be controlled by pressing a main body portion. An example of the drug administration device shown in FIGS. 63A and 63B includes a hollow main body portion 21 with a rectangular parallelepiped shape whose lower portion is open, and a movable portion 22 to which a needle member 4 is attached is accommodated in the main body portion 21. The movable portion 22 is held in the main body portion 21 so as to be capable of being moved up and down. Two opposing surfaces 211 and 212 of the main body portion 21 can be plastically deformed. The movable portion 22 is configured to be capable of being located at a first position where the needle member 4 is accommodated in the main body portion 21 as shown in FIG. 63A and at a second position where the needle member 4 protrudes from the lower opening of the main body portion 21 as shown in FIG. 63B.

When the movable portion 22 is located at the first position, the opposing surfaces 211 and 212 of the main body portion 21 are pressed against the movable portion 22, and thus the movable portion 22 is held at the first position. When the vicinities of the upper portions of the opposing surfaces 211 and 212 of the main body portion 21 are pressed together in this state and thus allowed to approach each other, the movable portion 22 is pushed down and is moved to the second position. At this time, the opposing surfaces 211 and 212 of the main body portion 21 approach each other above the movable portion 22 due to plastic deformation. Accordingly, the internal space of the main body portion 21 is reduced above the movable portion 22. Therefore, the upward movement of the movable portion 22 is restricted, and thus the movable portion 22 is held at the second position. Accordingly, by attaching the lower opening of the main body portion 21 to the skin in the state in which the movable portion 22 is located at the first position, and then pressing together the opposing surfaces 211 and 212 of the main body portion 21 in this state, it is possible to insert the needle member 4 into the skin.

### C-20

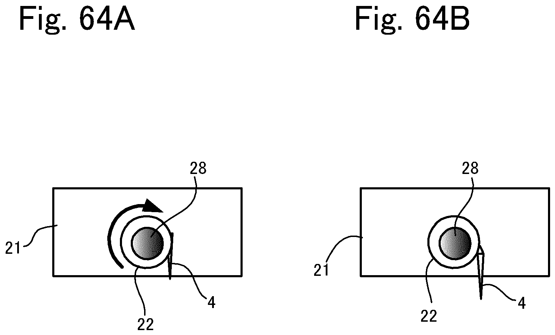

A means for moving a movable portion can be configured as shown in FIGS. 64A and 64B, for example. An example of the drug administration device shown in FIGS. 64A and 64B includes a main body portion 21 with a cylindrical tubular shape whose lower portion is open, and a movable portion 22 to which a needle member 4 is attached is accommodated in the main body portion 21. The movable portion 22 is rotatably held inside the main body portion 21, and a dial 28 for rotating the movable portion 22 is attached to the outer circumferential surface of the main body portion 21. Moreover, the movable portion 22 is provided with the needle member 4, and is configured to enter a state in which the needle member 4 is accommodated in the main body portion 21 as shown in FIG. 64A and a state in which the needle member 4 protrudes from the lower opening of the main body portion 21 as shown in FIG. 64B.

That is, a position of the movable portion 22 rotated until the needle member 4 is accommodated in the main body portion 21 is taken as a first position, and a position of the movable portion 22 rotated using the dial 28 from the first position until the needle member 4 protrudes from the main body portion 21 is taken as a second position. Accordingly, by attaching the lower opening of the main body portion 21 to the skin in the state in which the movable portion 22 is located at the first position, and then rotating the dial 28 in this state, it is possible to insert the needle member 4 into the skin.

It should be noted that, in the above-mentioned aspects of C-17 to C-20, there is no particular limitation on the configuration for supplying a drug to the needle member, and it is sufficient that a tubing member is connected to the movable portion or the needle member.

### C-21

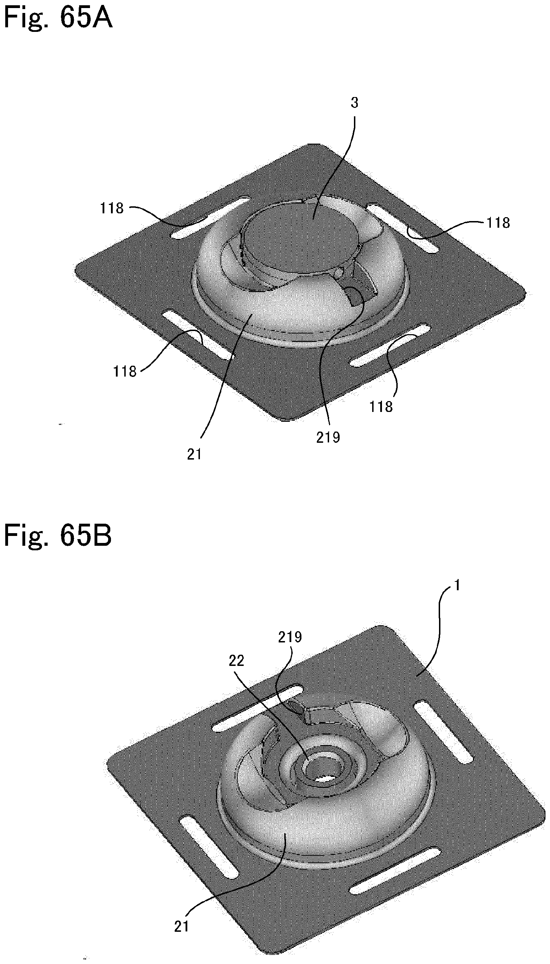

There is no particular limitation on the configuration of the main body portion, and a configuration as shown in FIGS. 65A and 65B can be employed, for example. FIG. 65A is a perspective view of a drug administration device, and FIG. 65B is a perspective view of the drug administration device from which an operation portion and a needle member are removed. The configuration of a main body portion 21 of the example shown in FIGS. 65A and 65B is different from that of the above-mentioned embodiment shown in FIG. 1. As shown in FIG. 65A, a configuration can be employed in which a movable portion 22 and an operation portion 3 are accommodated in the main body portion 21 when the movable portion 22 is located at a second position. That is, as shown in FIG. 65B, the volume of the circumferential edge portion of the main body portion 21 is increased, and the operation portion 3 is accommodated in the main body portion 21 when the movable portion 22 is located at the second position. Accordingly, the main body portion 21 surrounds the operation portion 3, and the upper surface of the operation portion 3 is substantially flush with the upper end portion of the main body portion 21, thus making it possible to accommodate the operation portion 3 located at the second position in the main body portion 21. It should be noted that, as shown in FIG. 65A, a configuration may also be employed in which the main body portion 21 is provided with a cutout portion 219 at a portion of the circumferential edge, and the side surface of the operation portion 3 is exposed to the outside through this cutout portion 219. Thus, a drug line (not shown) can be coupled to the exposed side surface of the operation portion 3.

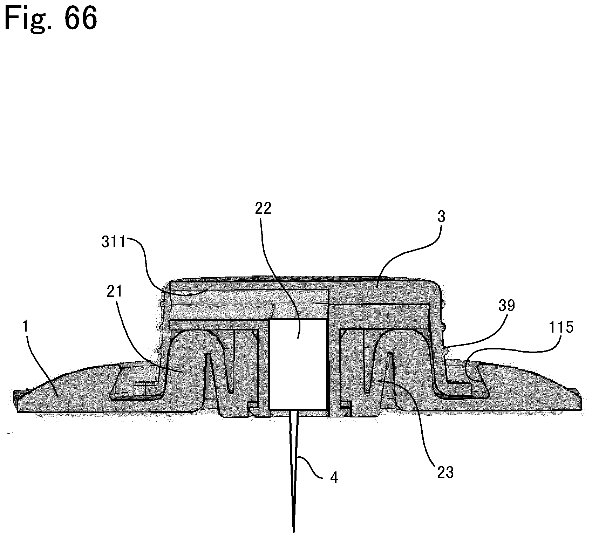

Moreover, as shown in FIG. 66, when the operation portion 3 is provided with an extension portion 39, a groove 115 in which the lower end of the extension portion 39 can be accommodated can also be formed in the upper surface of the base 1. This groove 115 makes it possible to prevent the extension portion 39, the movable portion 22, and the needle member 4 from being moved when an external force is applied to the extension portion 39. However, as shown in FIG. 66, a configuration can also be employed in which the extension portion 39 is not completely fixed to the groove 115, but is allowed to be slightly moved due to the application of an external force by setting the width of the groove 115 to be slightly larger than the width of the lower end portion of the extension portion 39. Accordingly, a force applied to the extension portion 39 can be absorbed, thus making it possible to suppress direct transmission of this force to the base 1. Thus, it is possible to suppress dislodgement of the base 1 from the skin.

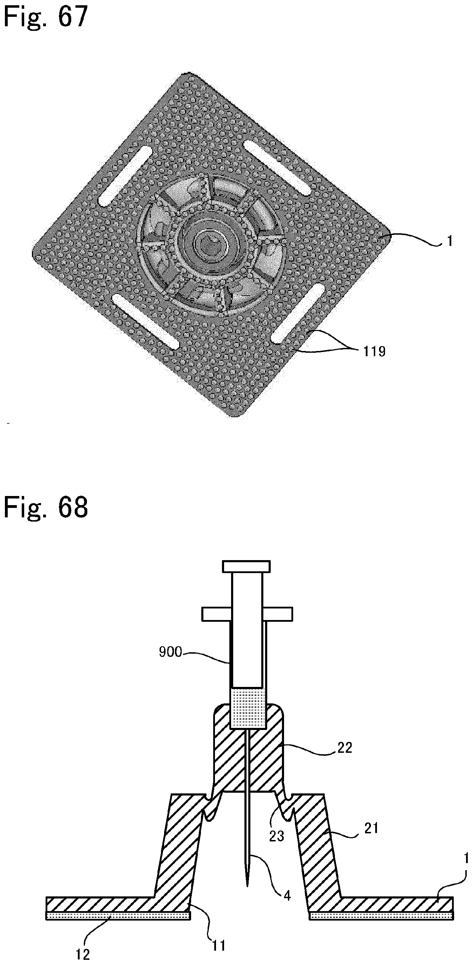

In the example shown in FIG. 65, the base 1 is formed in a rectangular shape and provided with slits 118 extending along the four sides of the base 1. These slits 118 are provided for the purpose of improving air permeability and enables sweating of the skin to which the base 1 is attached to be suppressed. Moreover, as shown in FIG. 67, a configuration may also be employed in which a large number of protrusions 119 are formed on the lower surface of the base 1, and thus a gap is formed between the lower surface of the base 1 and the skin. Alternatively, air permeability can also be improved by forming pores in portions other than portions on which the protrusions 119 are formed. In the case where the main body portion 21 is directly attached to the skin, such a configuration for improving air permeability can also be provided to the main body portion 21.

### C-22

A drug administration device as shown in FIG. 68 can also be configured as a method for administering a drug. In the example shown in FIG. 68, there is no particular limitation on the configurations of a base 1, a main body portion 21, a coupling portion 23, a movable portion 22, and a needle member 4, but they are the same as those shown in FIG. 22, for example. In addition thereto, a syringe 900 containing a drug is attached to the movable portion 22, and a discharge port of the syringe 900 and the needle member 4 are in communication with each other. Accordingly, by pressing the plunger of the syringe 900 after moving the movable portion 22 to the second position, the drug is discharged from the syringe 900 and administered to the skin through the needle member 4. Such a syringe 900 for supplying a drug can be applied to the other drug administration devices described above as appropriate. Note that this syringe 900 can be another example of the storage portion of the present invention. Note that this syringe 900 can be another example of the storage portion of the present invention.

### C-23

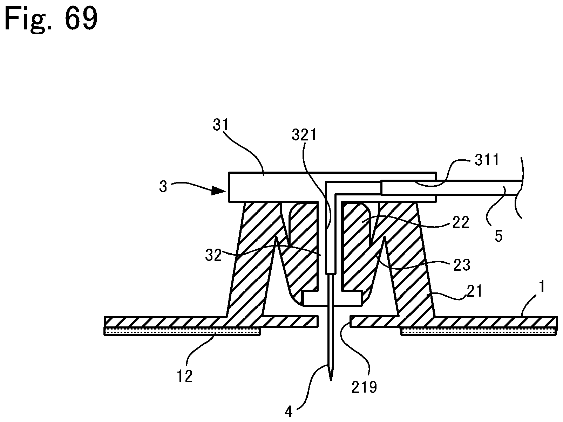

Although the main body portion 21 is provided with the internal space with a cylindrical tubular shape that is large enough for the movable portion 22 to be moved up and down in the example shown in FIG. 4, for example, a configuration as shown in FIG. 69 can also be employed in which the opening at the lower end portion of the main body portion 21 is partially blocked and thus a through hole 219 having such a size that allows the needle member 4 to pass therethrough is formed.

### C-24

The needle member 4 can also be configured to be capable of being rotated around the axis in the embodiments. For example, in a case where the needle member 4 is provided with a needle hole in the side surface or the leading end of the needle member 4 is obliquely cut, it is possible to change the position in the radial direction at which a liquid drug is discharged by rotating the needle member 4 around the axis. Thus, it is also possible to adjust the flow rate of the liquid. When such a configuration is employed, the operation portion 3 shown in FIG. 4 can be connected to the movable portion 22 such that they can be rotated around the axis of the needle member 4, for example. Thus, it is possible to change the position of the needle hole in the circumferential direction by rotating the operation portion 3 relative to the movable portion 22 after the insertion of the needle member 4. In order to enable the position of the needle hole to be changed as described above, it is necessary to fix the operation portion 3 with a piece of tape or the like so as to fix the operation portion 3 to the movable portion 22 at a desired position, or to employ a configuration in which the operation portion 3 can be held at a predetermined rotation position relative to the movable portion 22.

### C-25

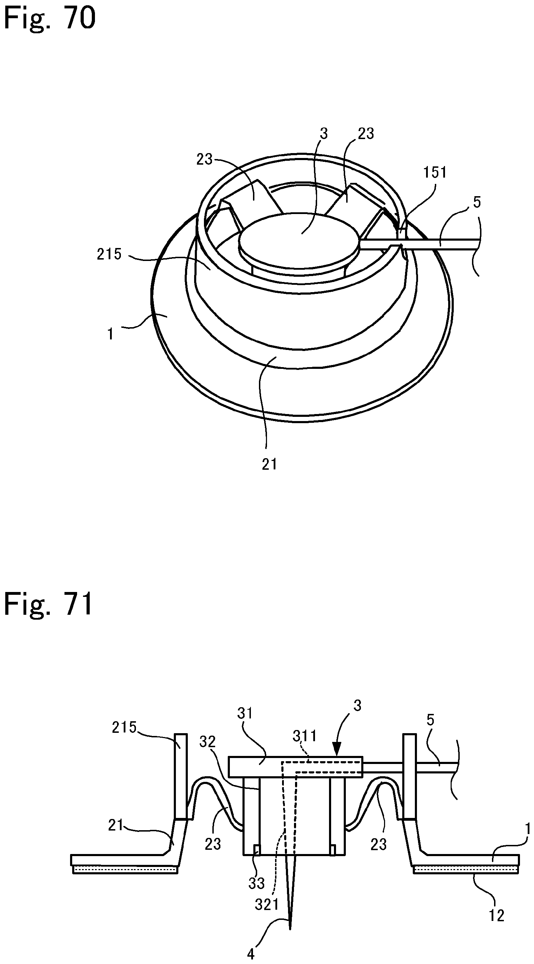

As shown in FIGS. 70 and 71, the main body portion 21 can also be provided with an accommodation portion 215 at the upper end such that the movable portion 22 and the operation portion 3 located at the second position are accommodated in the accommodation portion 215. The accommodation portion 215 is formed in a cylindrical tubular shape by extending the main body portion 21 upward. Providing such an accommodation portion 215 makes it possible to prevent the movable portion 22 and the operation portion 3 located at the second position from being caught on clothes or the like. It should be noted that the accommodation portion 215 can be provided with a cutout 151 at a position at which the drug line 5 is to pass through. It should be noted that there is no particular limitation on the position of the accommodation portion 215, and it is sufficient that the accommodation portion 215 is formed in a tubular shape such that the movable portion 22 and the operation portion 3 located at the second position are accommodated therein. Accordingly, the accommodation portion 215 can be provided at the upper end of the main body portion 21 as well as on the outside of the main body portion 21 in the radial direction. It should be noted that the example shown in FIGS. 70 and 71 is based on Second Embodiment, but this configuration may also be based on First Embodiment or can be applied to various examples described as the modified examples.

### LIST OF REFERENCE NUMERALS

- 1: Base
- 12: Adhering material
- 14: Recessed portion
- 2: Drug administration portion
- 21: Main body portion
- 215: Accommodation portion
- 22: Movable portion
- 23: Coupling portion
- 3: Operation portion
- 39: Extension portion
- 4: Needle member

## Claims

1. A drug administration device for subcutaneously administering a drug, comprising:
a main body portion configured to be arranged on skin of a patient; and
a movable portion to which at least one needle member protruding toward the skin is attached,
wherein the movable portion is configured to be capable of being moved between a first position that is spaced apart from the skin and a second position that is near the skin,
a leading end portion of the needle member is to be inserted into the skin when the movable portion is located at the second position, and
the drug is to be discharged from a hole provided in the needle member.

2. The drug administration device according to claim 1, wherein the movable portion is configured to be held at at least one of the first position and the second position in a state in which an external force is not applied to the movable portion.

3. The drug administration device according to claim 1 or 2, further comprising: at least one coupling portion that couples the main body portion and the movable portion to each other and is configured to be capable of holding the movable portion at at least one of the first position and the second position.

4. The drug administration device according to claim 3,
wherein a plurality of the coupling portions are provided, and
the coupling portions are constituted by a plurality of parts that are radially arranged around the needle member at predetermined intervals.

5. The drug administration device according to claim 4,
wherein the coupling portions are formed of a material that can be elastically deformed, and are configured to enter a first state when located at the first position and enter a second state when located at the second position,
the coupling portions in the second state are bent, and the coupling portions in the first state are more stretched than those in the second state, and
the coupling portions are configured to make a transition between the first state and the second state while being elastically deformed.

6. The drug administration device according to claim 4 or 5, wherein two to ten coupling portions are provided.

7. The drug administration device according to any one of claims 3 to 6,
wherein the main body portion, the movable portion, and the coupling portion are formed as a single body.

8. The drug administration device according to any one of claims 3 to 7,
wherein the main body portion, the movable portion, and the coupling portion are formed of an elastically deformable elastomer, hard resin, or metal.

9. The drug administration device according to any one of claims 1 to 8,
wherein the main body portion is formed in a tubular shape, and
the movable portion is configured such that at least a portion of the movable portion is accommodated in the main body portion when the movable portion is located at the second position.

10. The drug administration device according to any one of claims 1 to 9,
wherein a drug line for supplying the drug can be attached, and
the drug is supplied from the drug line to the needle member.

11. The drug administration device according to any one of claims 1 to 10, further comprising
an operation portion that is coupled to the movable portion and can be held by tools or fingers.

12. The drug administration device according to claim 11, wherein the operation portion is formed in a plate shape whose external size is larger than that of the main body portion.

13. The drug administration device according to claim 12, further comprising
an extension portion connected to the operation portion,
wherein the extension portion covers at least a portion of the main body portion when the movable portion is located at the second position.

14. The drug administration device according to claim 11 or 13,
wherein the operation portion is configured to protrude outward from the outer circumference of the movable portion in a radial direction, and
a stopper is further included that is detachably arranged between the operation portion and the main body portion when the movable portion is located at the first position, and restricts movement of the movable portion to the second position.

15. The drug administration device according to any one of claims 1 to 14, further comprising
a storage portion for storing the drug,
wherein the drug is supplied from the storage portion to the needle member.

16. The drug administration device according to any one of claims 1 to 15, further comprising a base that supports the main body portion and is to be arranged on the skin.

17. The drug administration device according to claim 16, further comprising an adhering material arranged on at least a portion of a surface of the base that is to be brought into contact with the skin.

18. The drug administration device according to claim 16 or 17, wherein a recessed portion is provided on a surface of a circumferential edge portion of the base that is to be opposite to the skin so as to form a gap formed between the base and the skin.

19. The drug administration device according to any one of claims 16 to 18,
wherein a plurality of drug administration portions are arranged on the base, and
each of the plurality of drug administration portions includes the main body portion, the movable portion, and the needle member.

20. The drug administration device according to claim 19, wherein all of the movable portions of the drug administration portions are coupled to each other.

21. The drug administration device according to any one of claims 3 to 8, further comprising
a base that supports the main body portion and is to be arranged on the skin,
wherein the base, the main body portion, the movable portion, and the coupling portion are formed of an elastomer as a single body.

22. The drug administration device according to any one of claims 1 to 15,
wherein the main body portion is configured to be attached to the skin, and
the main body portion is provided with a recessed portion so as to form a gap between the main body portion and the skin.

23. The drug administration device according to any one of claims 11 to 22, wherein the needle member is fixed to the operation portion, and the operation portion can be detached from the movable portion together with the needle member.

24. The drug administration device according to any one of claims 1 to 23, further comprising a detaching means for detaching the needle member from the movable portion.

25. The drug administration device according to any one of claims 1 to 24, further comprising
a cover member that can cover at least a leading end of the needle member,
wherein the leading end of the needle member is exposed from the cover member when the movable portion is located at the second position, and the cover member covers the leading end of the needle member and this covering state is maintained after the movable portion is moved from the second position to the first position.

26. The drug administration device according to claim 25, wherein, at the first position before use, the leading end of the needle member is covered by the cover member.

27. The drug administration device according to claim 25 or 26,
wherein the cover member is configured to be capable of being detached from the movable portion together with the needle member.

28. The drug administration device according to any one of claims 1 to 27, wherein the needle member has a thickness of a gauge of 30 to 35.

29. The drug administration device according to any one of claims 1 to 28, wherein a portion of the needle member protruding from a surface of the drug administration device that is to be fixed to the skin has a length of 1 to 10 mm.

30. The drug administration device according to any one of claims 1 to 29, further comprising an accommodation portion configured to accommodate at least the movable portion when the movable portion is located at the second position.

31. The drug administration device according to any one of claims 1 to 30, wherein the needle member is configured to be capable of being rotated around the axis of the needle member relative to the movable portion.

32. A drug administration system comprising:
at least one drug administration device according to any one of claims 1 to 31;
at least one drug container; and
a drug line for supplying a drug from the drug container to the drug administration device.

33. A drug administration system, comprising;
at least one drug administration device according to any one of claims 1 to 31; and
a syringe capable of supplying a drug to the needle member.
